(19) Europäisches Patentamt
European Patent Office
Office européen des brevets

(11) Veröffentlichungsnummer: **0 183 159**
**A2**

(12) **EUROPÄISCHE PATENTANMELDUNG**

(21) Anmeldenummer: 85114618.3

(22) Anmeldetag: 18.11.85

(51) Int. Cl.4: **A 61 K 31/44**
A 61 K 31/505, C 07 D 401/04
C 07 D 403/04

(30) Priorität: 28.11.84 DE 3443308

(43) Veröffentlichungstag der Anmeldung:
04.06.86 Patentblatt 86/23

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Sasse, Klaus, Dr.
Puetzweg 13
D-5060 Bergisch-Gladbach 2(DE)

(72) Erfinder: Hammond, Michael, Dr.
3 London Road
Postcombe Oxon(GB)

(72) Erfinder: Seuter, Friedel, Dr.
Moospfad 16
D-5600 Wuppertal 11(DE)

(72) Erfinder: Perzborn, Elisabeth, Dr.
Am Tescher Busch 13
D-5600 Wuppertal 11(DE)

(72) Erfinder: Pelster, Bernhard, Dr.
Pleisufer 6a
D-5205 St. Augustin 1(DE)

(72) Erfinder: Sturton, Graham, Dr.
45 Burnham Lane
Slough Bucks(GB)

(72) Erfinder: Abram, Trevor, Dr.
214 Marlow Bottom
Marlow Bucks(GB)

(54) **1-Heteroaryl-4-aryl-pyrazolin-5-one zur Verwendung als Arzneimittel.**

(57) Die Erfindung betrifft 1-Heteroraryl-4-aryl-pyrazolin-5-one, ein Herstellungsverfahren und die Verwendung bei der Bekämpfung von Krankheiten, insbesondere zur Verwendung als Lipoxygenasehemmer.

EP 0 183 159 A2

Croydon Printing Company Ltd.

BAYER AKTIENGESELLSCHAFT 5090 Leverkusen, Bayerwerk
Konzernverwaltung RP
Patentabteilung
Ad/by-c

# 1-Heteroaryl-4-aryl-pyrazolin-5-one zur Verwendung als Arzneimittel

Die vorliegende Erfindung betrifft 1-Heteroaryl-4-aryl-pyrazolin-5-one als Arzneimittel.

Die Erfindung betrifft auch die Verwendung von Pyrazolinonen als Hemmer/Stimulatoren von enzymatischen Reaktionen im Rahmen des Arachidonsäurestoffwechsels. Derartige Substanzen sind zur Verhütung und Behandlung von Erkrankungen der Atemwege wie Allergien/Asthma, Bronchitis, Emphysem, Schocklunge, pulmonaler Hypertonie, Entzündungen/Rheuma, Arthrosen und Ödemen, Thrombosen und Thromboembolien, Ischämien (periphere, kardiale, cerebrale Durchblutungsstörungen), Herz- und Hirninfarkten, Herzrhythmusstörungen, Angina pectoris, Arteriosklerose, bei Gewebetransplantationen, Dermatosen wie Psoriasis, Metastasen und zur Cytoprotektion im Gastrointestinaltrakt geeignet.

Gegenstand der Erfindung sind auch Arzneimittel, welche durch den Gehalt an einer therapeutisch wirksamen Menge von Pyrazolinonen gekennzeichnet sind.

Die erfindungsgemäßen Verbindungen sind bevorzugt Hemmer der Lipoxygenase und stimulieren gleichzeitig die Prostacyclinsynthese. Bekannte Lipoxygenasehemmer wie Nordihydroguaretsäure, 3-Amino-1-(3-trifluormethylphenyl)-pyrazolin (BW 755C), Phenidon und 5,8,11,14-Eikosatetrainsäure sind entweder gleichzeitig als Cyclooxygenasehemmer oder erst bei sehr hohen Konzentrationen wirksam. Die Hemmung des Enzyms Cyclooxygenase des Arachidonsäuremetabolismus führt zu einer globalen Prostaglandinsynthesehemmung und im allgemeinen zu einer Stimulation des Lipoxygenaseweges, was Gastrotoxizität und pro-inflammatorische und asthmatische Wirkungen sowie eine erhöhte Thrombose- und Arterioskleroseneigung verursachen kann.

Außerdem haben bekannte Lipoxygenasehemmer wie 3-Amino-1-(m-trifluormethylphenyl)-pyrazolin-2 bei systemischer Verabreichung (z. B. oral) toxische Nebenwirkungen. Es besteht deshalb ein Bedarf nach stärker wirksamen Verbindungen mit einem selektiveren Wirkungsprofil ohne Nebenwirkungen.

Überraschenderweise entsprechen die erfindungsgemäßen Pyrazolinone dem geforderten pharmakologischen Profil.

Seit der Entdeckung der "slow reacting substance of anaphylaxis (SRS-A)" wurde ihr eine wichtige Rolle bei asthmatischen Bronchokonstriktionen und bei der Entzündung zugeschrieben. Sie wird über den Lipoxygenaseweg des Arachidonsäurestoffwechsels gebildet und als Leukotrien (e)

identifiziert ($LTD_4$, $LTC_4$, $LTE_4$). Entsprechende Inhibitoren sind daher potentielle Antiasthmatika. Die bisher bekannt gewordenen Verbindungen erfüllen jedoch nicht die Forderungen, bzw. sind therapeutisch nicht verwendbar.

Es wurden Pyrazolinone zur Verwendung als Arzneimittel der Formel

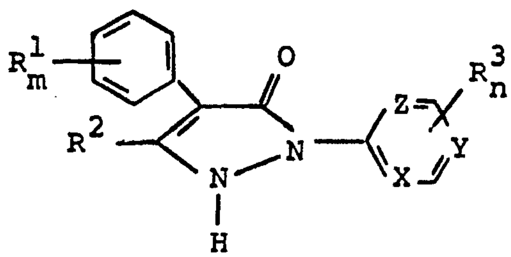 (I),

gefunden

in der

$R^1$ Wasserstoff, Halogen, Hydroxy, gegebenenfalls substituiertes Niederalkoxy, gegebenengalls substituiertes Phenoxy, gegebenenfalls substituiertes Niederalkylmercapto, gegebenenfalls substituiertes Niederalkylsulfonyl, gegebenenfalls durch Fluor substituiertes Niederalkyl, einen ankondensierten carbocyclischen oder heterocyclischen Rest, Carboxyl, Niederalkoxycarbonyl oder eine der Gruppen

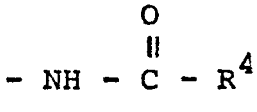 , 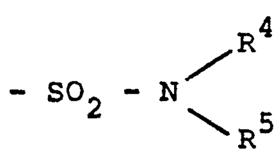 ,

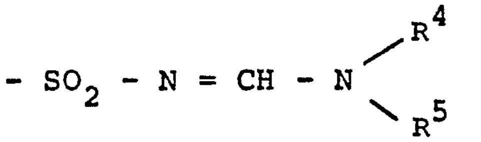 , 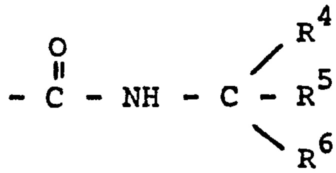

oder

$$-\overset{O}{\overset{\|}{C}}-NH-R^4$$

bedeutet

wobei

$R^4$, $R^5$ und $R^6$ gleich oder verschieden sind und Wasserstoff, Niederalkyl oder Phenyl bedeuten,

m eine ganze Zahl von 1 bis 5 bedeutet,

$R^2$ Wasserstoff oder Niederalkyl bedeutet,

X, Y und Z ein N-Atom oder ein Ringglied $=\overset{R^3}{\overset{|}{C}}-$ bedeuten, wobei wenigstens einer der X, Y oder Z ein N-Atom bedeutet,

$R^3$ Wasserstoff, Halogen, Niederalkoxy, Niederalkylmercapto, Niederalkyl, Niederhalogenalkyl, Nitro, Cyan, Carbonsäureamid oder einen ankondensierten Phenylenrest bedeutet, und

n eine ganze Zahl von 1 bis 4 bedeutet.

Die erfindungsgemäßen Pyrazolinone können in tautomeren Formen vorliegen:

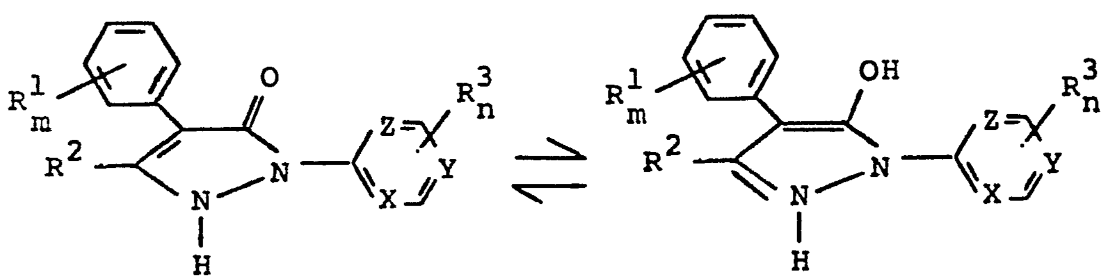

Im Rahmen der vorliegenden Erfindung können die Substienten im allgemeinen die folgende Bedeutung haben:
Halogen bedeutet im allgemeinen Fluor, Chlor, Brom oder Iod, bevorzugt Fluor und Chlor.

Niederalkyl bedeutet im allgemeinen einen geradkettigen oder verzweigten Kohlenwasserstoffrest mit 1 bis etwa 6

Kohlenstoffatomen. Beispielsweise seien die folgenden Niederalkylreste genannt: Methyl, Ethyl, Propyl, Isopropyl, Butyl, Isobutyl, Pentyl, Isopentyl, Hexyl und Isohexyl. Bevorzugt sind der Methyl- und Ethylrest.

Niederalkoxy bedeutet im allgemeinen einen über Sauerstoff gebundenen geradkettigen oder verzweigten Kohlenwasserstoffrest mit 1 bis etwa 6 Kohlenstoffatomen. Beispielsweise seien die folgenden Niederalkoxyreste genannt: Methoxy, Ethoxy, Propoxy, Isopropoxy, Butoxy, Isobutoxy, Pentoxy, Isopentoxy, Hexoxy und Isohexoxy. Bevorzugt sind der Methoxy- und der Ethoxyrest.

In den Niederalkylmercapto, Niederalkylsulfonyl und Niederalkylesterresten hat der Niederalkylrest im allgemeinen den oben angegebenen Bedeutungsumfang.

Als mögliche Substituenten seien beispielsweise die folgenden Reste genannt:
Methylmercapto, Isopropylmercapto, Trifluormethylmercapto, Methylsulfonyl, Ethylsulfonyl, Butylsulfonyl, Methoxycarbonyl, Ethoxycarbonyl, tert.-Butoxycarbonyl

Der Rest $R^1$ kann für einen carbocyclischen oder heterocyclischen Rest stehen, der an den Phenylkern ankonsiert ist. Im allgemeinen handelt es sich um 5- oder 6-gliedrige Ringe, die außer Kohlenwasserstoffgliedern noch ein oder zwei Heteroatome, bevorzugt Stickstoff, Sauerstoff oder Schwefel, enthalten können. Beispielsweise seien die folgenden carbocyclischen oder heterocyclischen Ringe genannt:

Benzol, Furan, Thiophen, Pyrrol, Dioxolen, Dioxen, Pyridin, Pyrimidin, Pyrazin, Pyridazin, Imidazol, Triazol, Oxazol, Thiazol, Thiadiazol etc.

Bevorzugt werden Pyrazolinone der Formel

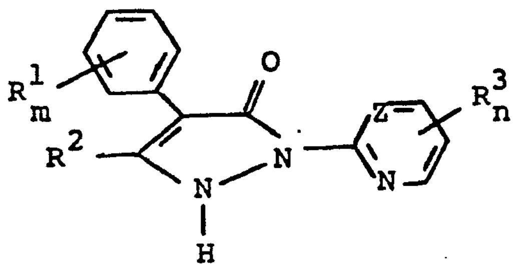

(II),

in der

$R^1$ Wasserstoff, Fluor, Chlor, Methyl, Ethyl, Methoxy, Ethoxy, Trifluormethyl, Trifluormethoxy oder Trifluormethylmercapto bedeutet,

m für die Zahl 1 oder 2 steht,

$R^2$ Wasserstoff, Methyl oder Ethyl bedeutet,

$R^3$ Wasserstoff, Fluor, Chlor, Methyl, Ethyl, Methoxy oder Ethoxy bedeutet und

n für die Zahl 1 oder 2 steht.

Im einzelnen seien die folgenden bevorzugten Pyrazolinone genannt:

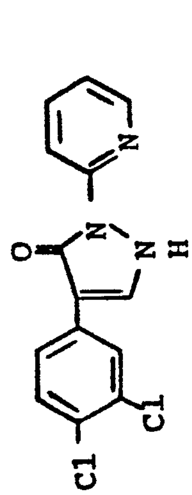

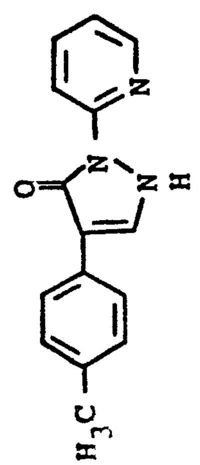

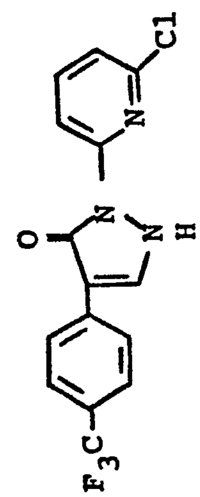

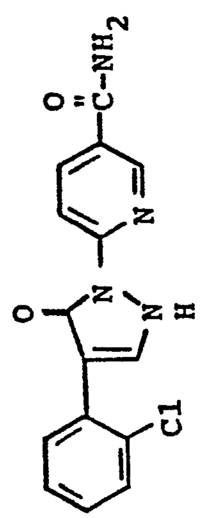

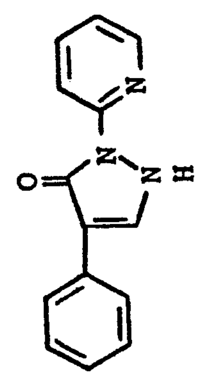

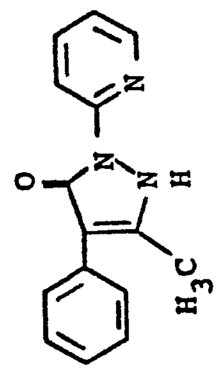

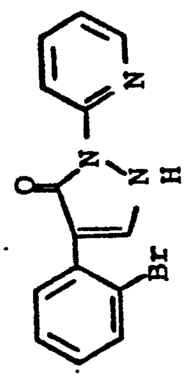

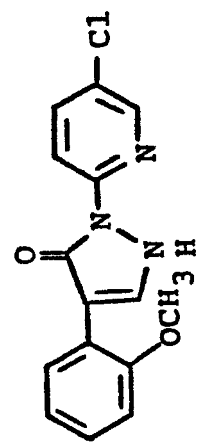

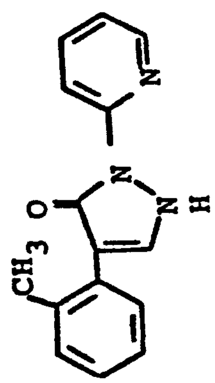

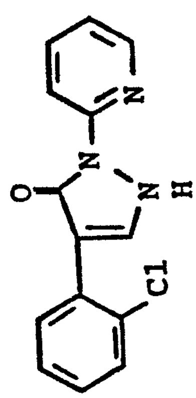

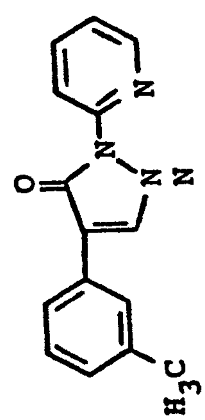

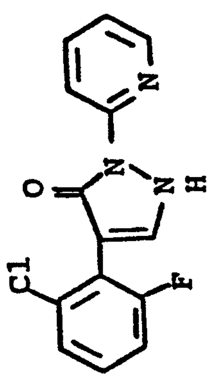

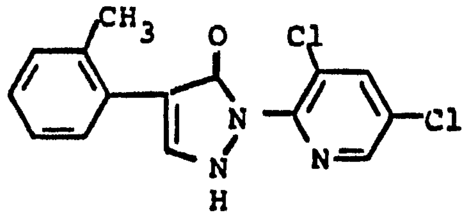

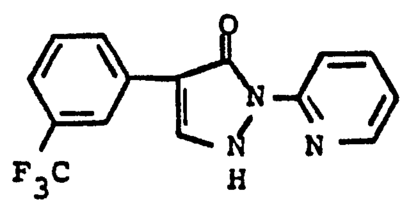

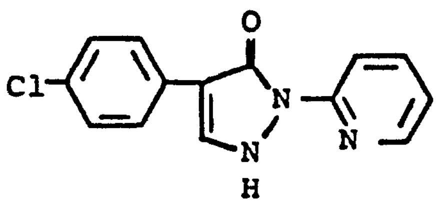

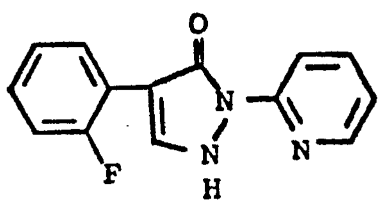

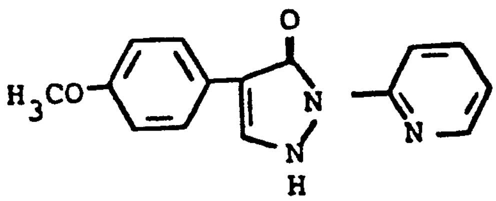

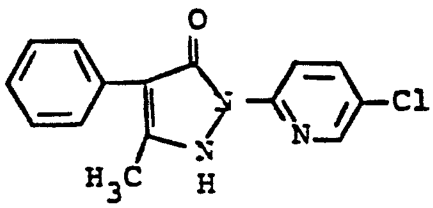

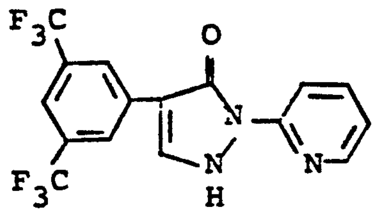

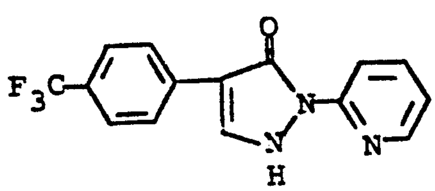

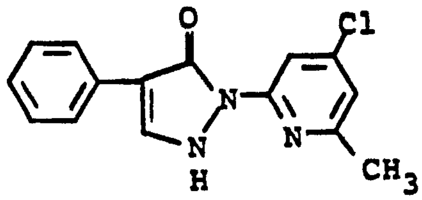

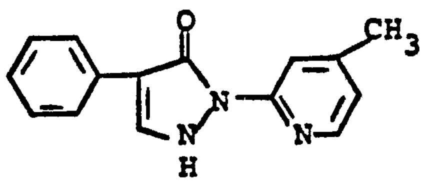

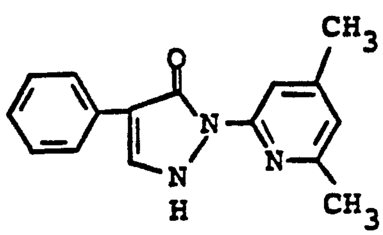

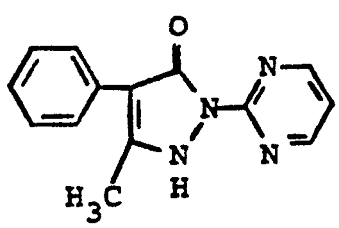

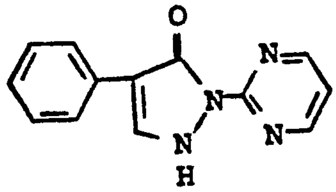

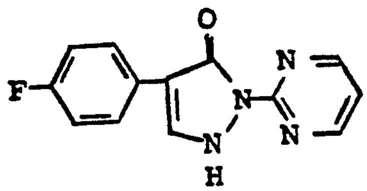

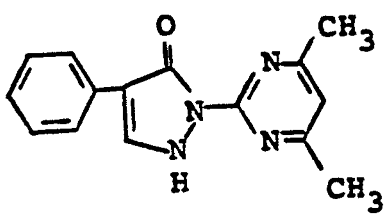

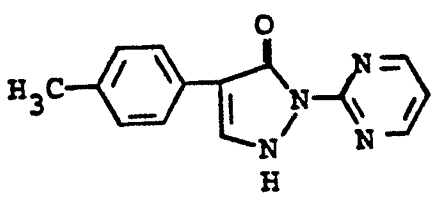

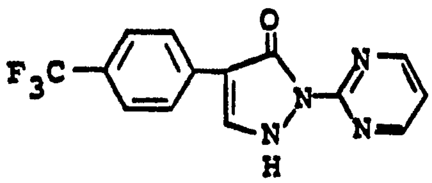

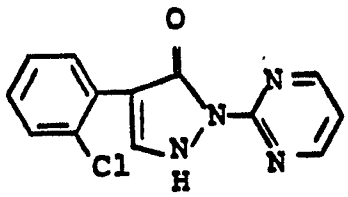

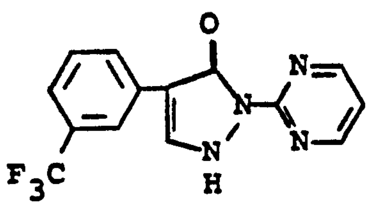

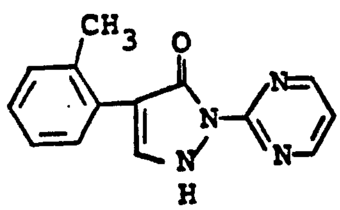

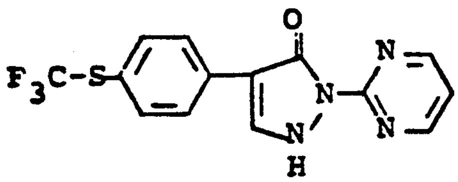

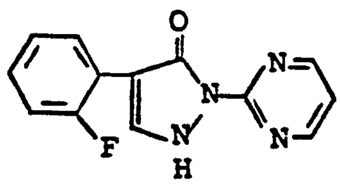

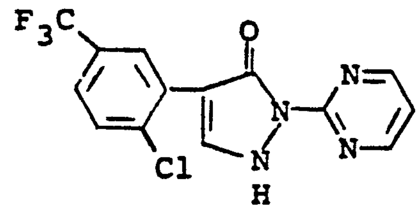

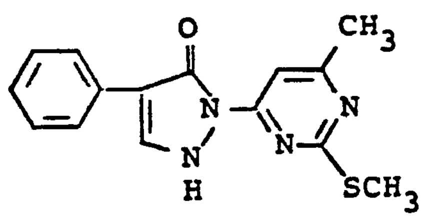

Die erfindungsgemäßen Verbindungen der Formel I können hergestellt werden, indem α-Acyl-phenylessigsäureester oder deren Derivate der Formel III mit Hydrazino-heterocyclen der Formel IV umgesetzt werden.

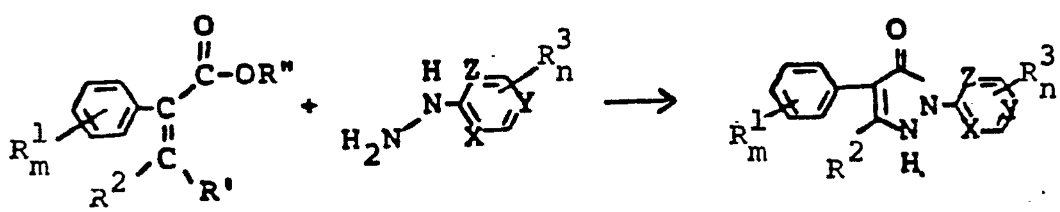

III IV I

In Formel III und IV haben $R^1$, $R^2$, $R^3$, X, Y, Z, n und m die gleiche Bedeutung wie in Formel I. R' steht für eine OH-Gruppe, eine niedere Alkoxygruppe, Halogen, eine R"'-$SO_2$-O-Gruppe oder für eine Dialkylaminogruppe und R" für einen niederen Alkylrest oder einen Arylrest.

Die zur Herstellung der erfindungsgemäßen Verbindungen der Formel I benötigten Vorprodukte der Formel III sind grundsätzlich bekannt. Für den Fall, daß R ein Wasserstoffatom darstellt, gewinnt man diese aus entsprechend substituierten Phenylessigsäurealkylestern durch Umsetzung mit einem Ameisensäurederivat. So werden Verbindungen III mit R' = OH hergestellt durch Einwirkung von Ameisensäure-methyl- oder -ethylester in Gegenwart starker Basen wie Alkalialkoholate, Natriumamid oder dergleichen (B. 20, 2931 (1887); B. 28, 771 (1895); Ann. 291, 164 (1896)), wobei zunächst die Alkalisalze (III; R' = O-Met.) entstehen, die auch direkt in die Folgereaktion eingesetzt werden können oder aber durch Behandeln mit wäßrigen Säuren in die freien Hydroxymethylenverbindungen übergehen, die im tautomeren Gleichgewicht mit den entsprechenden α-Formyl-phenylessigestern stehen.

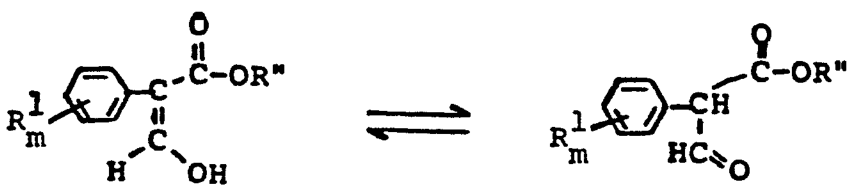

IIIa

Verbindungen mit R' = OAlk werden literaturgemäß hergestellt durch Einwirkung von Alkylierungsmitteln auf Verbindungen III mit R' = OH unter basischen Bedingungen (Ann. 424, 228 (1921); J. Chem. Soc. 1953, 3548; J. org. Chem. 45, 2576 (1980)) oder durch Veretherung der gleichen Verbindungen mit Alkoholen in Gegenwart von p-Toluol-sulfonsäure (J. chem. Soc. 1953, 3548).

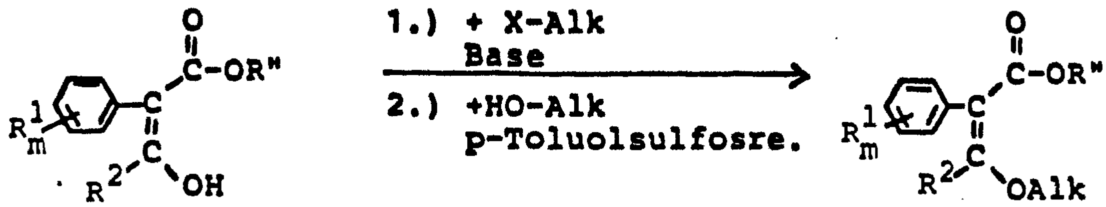


IIIa IIIb

Verbindungen III mit R' = R"'-$SO_2$-O-Gruppe lassen sich aus den entsprechenden Verbindungen III mit R' = OH durch Umsetzung mit Sulfonsäurechloriden, wie Methansulfochlorid, Trifluormethansulfochlorid oder p-Toluolsulfonsäurechlorid in Gegenwart von Alkalien herstellen:

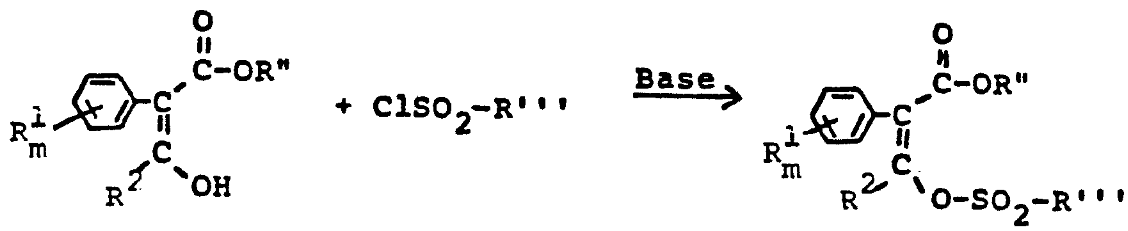


IIIc

Verbindungen III mit R' = Hal werden gewonnen, indem die Hydroxymethylenverbindungen (III) R' = OH) mit anorganischen Säurechloriden, vorzugsweise Phosphor(V)chlorid umgesetzt werden (B. 51, 1366 (1918)).

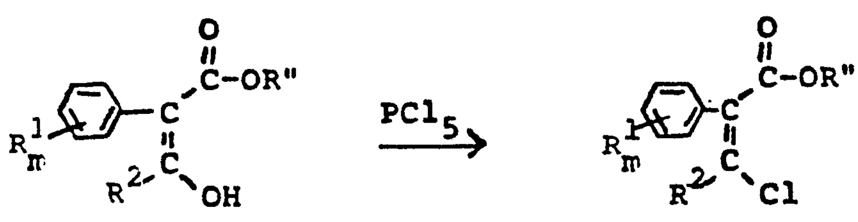


IIIa IIId

Verbindungen III mit R' = Dialkylamino werden gewonnen, indem die entsprechend substituierten Phenylessigsäurealkylester mit Dialkylformamid-dialkylacetalen umgesetzt werden (Tetrahedron Lett. <u>16</u>, 1361 (1979)), z.B.

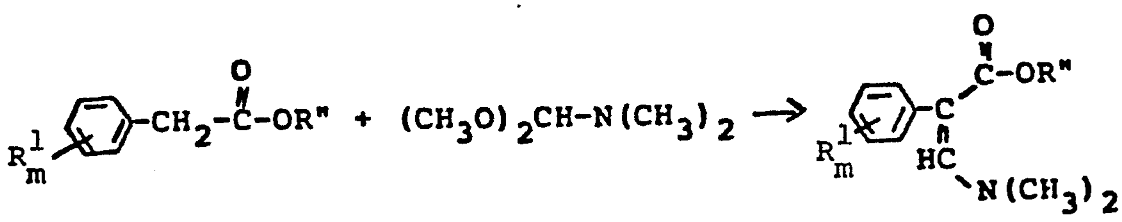

IIIe

Eine weitere Methode zur Herstellung dieser Verbindungen (IIIe) besteht in der Umsetzung der Hydroxymethylenderivate (IIIa) mit sekundären Aminen (A. ch (10) <u>18</u>, 103, 114 (1932)).

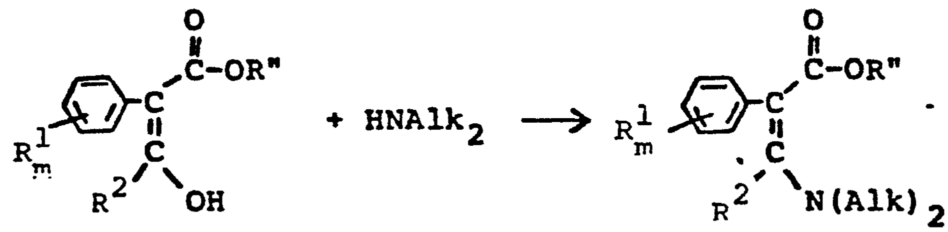

IIIa IIIe

Zur Herstellung der Verbindungen der Formel III, in denen $R^2$ einen niederen Alkylrest darstellt, lassen sich prinzipiell die gleichen Methoden anwenden wie zur Herstellung der Verbindungen mit $R^2$ = H, jedoch verlaufen diese häufig in unzureichenden Ausbeuten. Ergiebiger verläuft deren Synthese, wenn man entsprechend den Literaturangaben die Acylierung nicht mit den Phenylessigsäureestern, sondern mit Phenylacetonitrilen durchführt und nachträglich die Nitrilgruppe in die Estergruppe umwandelt:

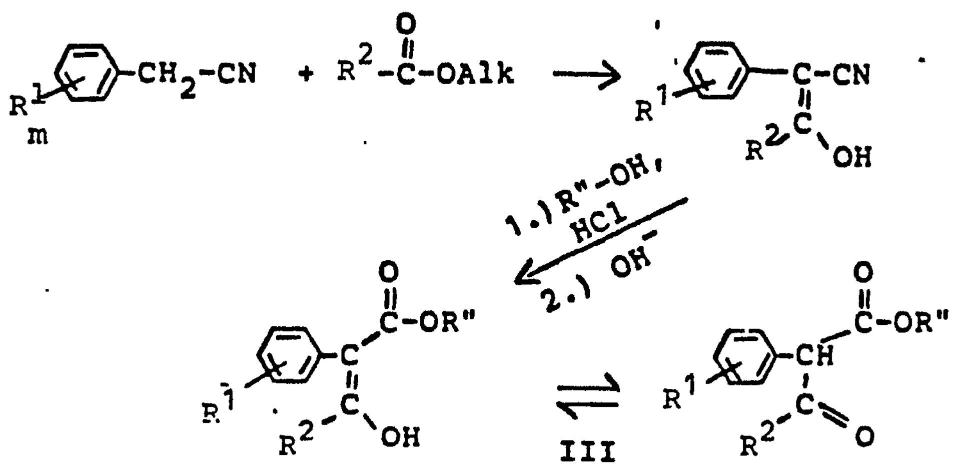


III

Die zur Herstellung der erfindungsgemäßen Verbindungen der Formel I benötigten Vorprodukte der Formel IV sind grundsätzlich bekannt. Es handelt sich um Hydrazinopyridine, Hydrazino-pyrimidine und Hydrazino-1,3,5-triazine:

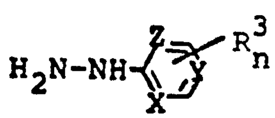

IV

In Formel IV bedeuten X, Y und Z ein N-Atom oder ein Ringglied -CH= oder $-\underset{R^3}{C}=$, wobei mindestens eines der X, Y und Z ein N-Atom bedeutet,

$R^3$ ein Wasserstoffatom, Halogen, insbesondere ein Chlor-, Brom- oder Fluoratom, eine niedere Alkylgruppe mit 1 bis 4 Kohlenstoffatomen, die auch substituiert sein kann, z.B. durch Halogen, insbesondere Fluor oder Chlor, oder durch eine niedere Alkoxy- oder Alkylmercaptogruppe mit 1 bis 4 Kohlenstoffatomen, eine Nitrogruppe, eine Cyangruppe

oder eine Carbonsäureamidgruppe. Der Substituent $R^3$ kann gemeinsam mit einem der benachbarten X, Y, Z, soweit es sich bei diesem um ein Kohlenstoff-Ringglied handelt, auch einen ankondensierten Ring, z.B. einen Phenylenring bilden.
n bedeutet eine ganze Zahl von 0 bis 4, jedoch höchstens eine Zahl, die der Anzahl der Kohlenstoffatome in dem heterocyclischen Ring entspricht.

Die Herstellung der Verbindungen IV ist grundsätzlich bekannt. Bisher nicht vorbeschriebene Varianten von IV lassen sich auf analogen Wegen herstellen, insbesondere

a) durch Umsetzung von Halogen-N-heterocyclen, Alkoxy- oder Alkylmercapto-N-heterocyclen mit Hydrazin:

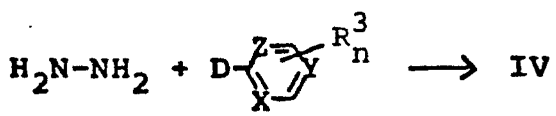

D= Hal, OAlk, SAlk

b) durch Reduktion von heterocyclischen Diazoniumverbindungen:

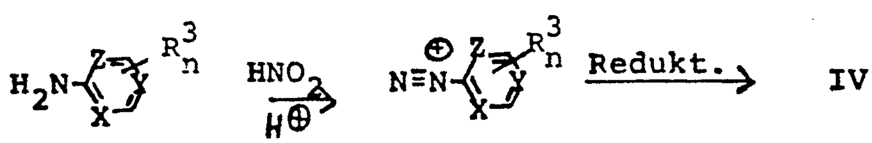

c) durch Reduktion von heterocyclischen Nitraminoverbindungen:

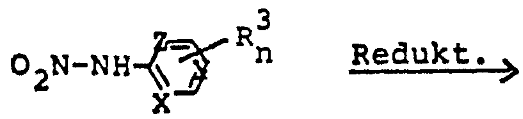


IV

Als Beispiele für Zwischenprodukte der Formel IV seien genannt:

| | Fp(°C)/Kp. | Literatur |
|---|---|---|
| 2-Hydrazino-pyridin | $Kp_{12}$: 130 | J. Chem.Soc. 107, 691 (1915) |
| 5-Chlor-2-hydrazino-pyridin | 127-128 | |
| 6-Chlor-2-hydrazino-pyridin | 116-117 | |
| 3,5-Dichlor-2-hydrazino-pyridin | 172-174 | |
| 5-Brom-2-hydrazino-pyridin | 133-134 | |
| 3-Nitro-2-hydrazino-pyridin | 200 | B. 57, 1192 (1924) |
| 5-Nitro-2-hydrazino-pyridin | 204 (Z.) | E.P. 2471488 |
| 5-Cyan-2-hydrazino-pyridin | 185-186 | |
| 2-Hydrazino-4-methyl-pyridin | 74-75 | Ann. 656, 103 (1962) |
| 2-Hydrazino-6-methyl-pyridin | HCl-Salz: 194 (Z.) | |
| 4-Chlor-2-hydrazino-6-methyl-pyridin | 162-163 | |
| 3-Nitro-2-hydrazino-6-methyl-pyridin | 138-139 | J. am. Chem. Soc. 74, 3828 (1952) |
| 5-Nitro-2-hydrazino-6-methyl-pyridin | 119-121 | J. am. Chem. Soc. 74, 3828 (1952) |
| 2-Hydrazino-4,6-dimethyl-pyridin | | |
| 3-Cyano-2-hydrazino-6-methyl-pyridin | 243 (Z.) | |
| 2-Hydrazino-5-trifluormethyl-pyridin | 68-69 | |
| 4-Hydrazino-pyridin | $Kp_{18}$:187 | B. 59, 317 (1926) |
| 4-Hydrazino-2,6-dimethyl-pyridin | | B. 31, 2497 (1898) |
| 3-Chlor-4-hydrazino-pyridin | | |
| 2-Hydrazino-chinolin | 142-143 | J. Chem. Soc. 103 1978 (1913) |

| | Fp(°C)/Kp. | Literatur |
|---|---|---|
| 2-Hydrazino-4-methyl-chinolin | 145-146 | |
| 4-Hydrazino-chinolin | | |
| 1-Hydrazino-isochinolin | 172 | Ann. 656, 103 (1962) |
| 2-Hydrazino-pyrimidin | 110-111 | |
| 5-Fluor-2-hydrazino-pyrimidin | 140-141 | |
| 5-Chlor-2-hydrazino-pyrimidin | 183-184 | |
| 4-Methoxy-2-hydrazino-pyrimidin | | |
| 2-Hydrazino-4-methyl-pyrimidin | 85-86 | |
| 2-Hydrazino-4,6-dimethyl-pyrimidin | 165 | J. chem. Soc. 1952, 4691 |
| 4-Methoxy-2-hydrazino-6-methyl-pyrimidin | | |
| 4-Hydrazino-pyrimidin | | |
| 4-Hydrazino-5-methyl-pyrimidin | 205-206 | Bl. Soc. chim. Belg. 68, 30, 32 (1959) |
| 4-Hydrazino-6-methyl-pyrimidin | 140-141 | B. 34, 1241 (1901) |
| 4-Hydrazino-2,6-dimethyl-pyrimidin | 192-193 | Bl. Soc. chim. Belg. 68, 30, 32 (1959) |
| 6-Chlor-4-hydrazino-2-methyl-pyrimidin | 152 (Z) | |
| 2-Methoxy-4-hydrazino-6-methyl-pyrimidin | | |
| 5-Methoxy-4-hydrazino-2-methyl-pyrimidin | | |
| 5-Methoxy-4-hydrazino-2-tert.butyl-pyrimidin | 213-214 | |
| 2-Methylmercapto-4-hydrazino-pyrimidin | 143-144 | |
| 2-Methylmercapto-4-hydrazino-6-methyl-pyrimidin | 142-143 | |

| | Fp(°C)/Kp. | Literatur |
|---|---|---|
| 2-Hydrazino-4,6-dimethyl-1,3,5-triazin | | |
| 4-Methoxy-2-hydrazino-6-methyl-1,3,5-triazin | | |
| 4-Methylmercapto-2-hydrazino-6-methyl-1,3,5-triazin | | |
| 4,6-Dimethoxy-2-hydrazino-1,3,5-triazin | 121 | |

Die Umsetzung der substituierten Atropasäureester der Formel III mit den Hydrazino-N-heterocyclen der Formel IV zu den erfindungsgemäßen 1-Heteroaryl-4-aryl-pyrazolin-5-onen der Formel I vollzieht sich in zwei Stufen, indem zunächst die endständige $NH_2$-Gruppe des heterocyclischen Hydrazins mit der Enol-, Enamin- bzw. Halogenvinylgruppe in III zu den En-hydrazinen bzw. Hydrazonen Ia in Reaktion tritt und nachfolgend unter Abspaltung des in III gebundenen Alkohols oder Phenols der Ringschluß zu I erfolgt.

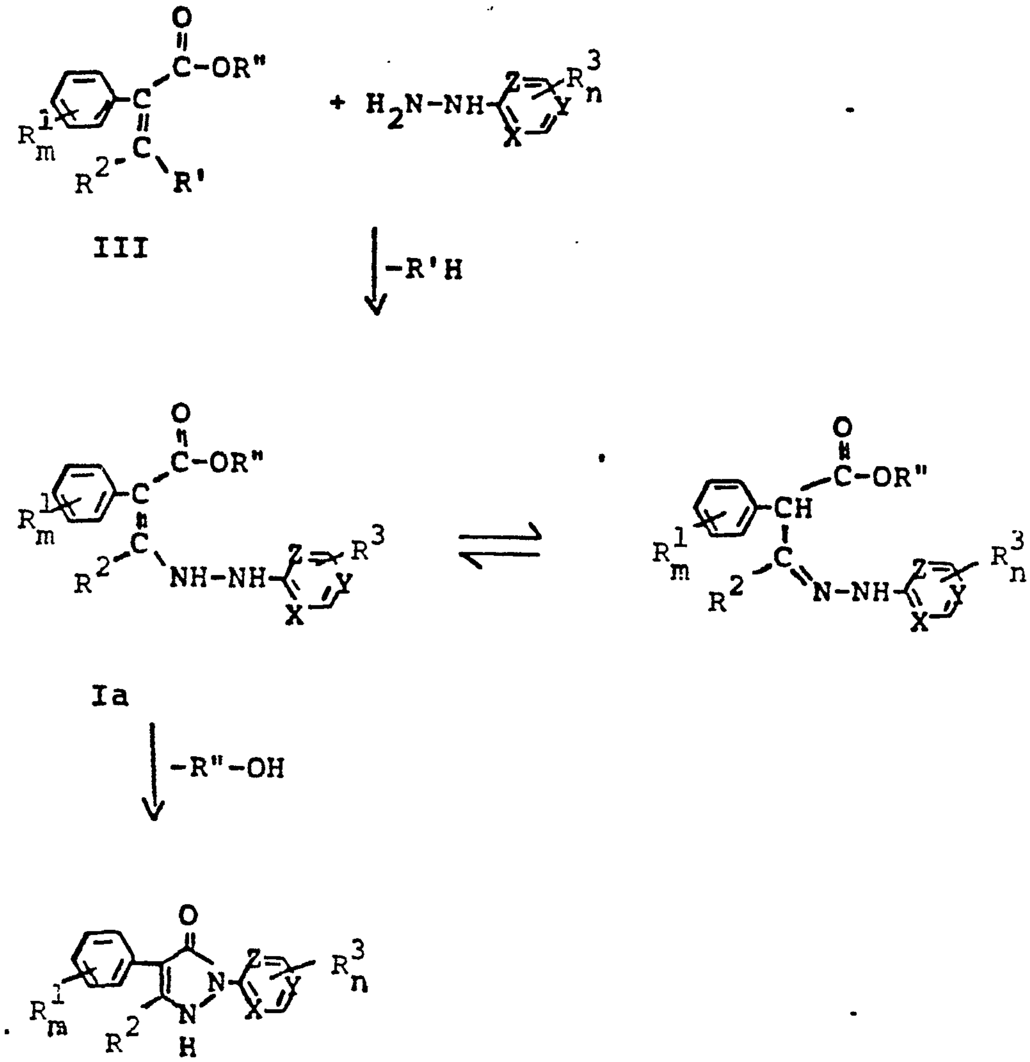


III

Ia

I

Die Zwischenstufen Ia können isoliert und in reiner Form in die Folgereaktion eingesetzt werden. Zweckmäßigerweise führt man aber beide Stufen gleichzeitig oder nacheinander im gleichen Ansatz durch.

Die Umsetzung der Vorprodukte III und IV miteinander zu den erfindungsgemäßen Verbindungen der Formel I kann ohne Lösungsmittel durch Erhitzen der Komponenten auf Temperaturen zwischen 50 und 150°C erfolgen.

Zweckmäßigerweise nimmt man die Reaktionen in Verdünnungsmitteln vor, wobei alle gegenüber den Reaktionspartnern inerte Lösungsmittel verwendet werden können. Hierzu gehören Kohlenwasserstoffe wie Benzin oder Toluol, Halogen-Kohlenwasserstoffe wie Di-, Tri- und Tetrachlormethan, Alkohole wie Methanol, Ethanol, Isopropanol und dergleichen, Ether wie Diethylether, Tetrahydrofuran oder Dioxan, Dimethylsulfoxid, Tetrahydrothiophendioxid, Dimethylformamid. Die Umsetzungen können auch in Wasser oder in Gemischen der genannten Lösungsmittel mit Wasser durchgeführt werden.

Die Reaktionstemperaturen können in einem größeren Bereich variiert werden. Im allgemeinen arbeitet man zwischen etwa 0 und 100°C, vorzugsweise zwischen Raumtemperatur und dem Siedepunkt des verwendeten Lösungsmittels.

In vielen Fällen entstehen die Verbindungen I aus III und IV unter den genannten Bedingungen ohne weitere Zusätze. In Abhängigkeit von der Abgangsgruppe R' in III

empfiehlt sich aber häufig das Arbeiten unter Zusatz von Basen oder Säuren. Der Zusatz von Basen ist zweckmäßig, wenn R' eine säureliefernde Abgangsgruppe darstellt, z.B. ein Halogenatom wie in IIId oder eine R"'-$SO_2$-O-Gruppe wie in IIIc. In diesen Fällen wendet man vorzugsweise die äquimolare Menge einer Base an. Als solche sind geeignet Alkali- und Erdalkalihydroxide und -carbonate, Alkoholate oder tertiäre Amine wie Triethylamin, Pyridin und dergleichen.

Der Zusatz von Säuren ist zweckmäßig, wenn die Abgangsgruppe R' eine basenliefernde Abgangsgruppe darstellt, z.B. eine Dialkylaminogruppe wie in IIIe. In diesen Fällen wendet man vorzugsweise die äquimolare Menge einer Säure an, z.B. Mineralsäuren wie Chlorwasserstoff oder Schwefelsäure oder eine organische Säure wie Essigsäure. Man kann hierbei auch so vorgehen, daß man die Enamine IIIb, z.B. durch Behandeln mit der äquimolaren Menge trockenem Chlorwasserstoff in inerten Lösungsmitteln in ein Salz überführt und diese zum Einsatz bringt.

Das Arbeiten im sauren Medium kann aber auch beim Einsatz von Verbindungen der Formel III mit R' = OH (IIIa) oder R' = OAlk zweckmäßig sein, wobei in der Regel untermolare Mengen von 0,1 bis 0,2 Mol genügen. Anstelle des nachträglichen Zusatzes von Säure kann man jedoch auch so vorgehen, daß man die Hydrazinoheterocyclen der Formel IV nicht als freie Basen sondern in Form ihrer Salze, z.B. der Hydrochloride in die Reaktion

einbringt. Diese Vorgehensweise ist insbesondere dann empfehlenswert, wenn die Hydrazine, wie das 2-Hydrazino-pyridin und das 4-Hydrazino-pyridin als freie Basen zur Zersetzung neigen.

Beim Arbeiten im sauren Medium bleibt die Reaktion in der Regel auf der nicht cyclisierten Stufe Ia stehen. Für den Ringschluß zu den erfindungsgemäßen Verbindungen I ist ein neutrales, günstigerweise basisches Medium notwendig. Falls die erste Stufe der Reaktion im sauren Medium durchgeführt wird, ist daher der Zusatz einer Base erforderlich. Als solche kommen vorzugsweise in Betracht: Alkali- und Erdalkali-hydroxide und -carbonate, sowie Alkali- und Erdalkali-alkoholate, sowie Alkaliamide (vorzugsweise beim Arbeiten in wasserfreien Medium).

Die Base wird mindestens in der zur Säuremenge äquivalenten Menge angewandt. Ein Überschuß bis zu einem weiteren Mol kann zweckmäßig sein, jedoch sollte das Medium einem pH-Wert von mindestens 9 entsprechen. Die Cyclisierungsstufe (Ia ⟶ I) wird bei einer Temperatur zwischen 0° und dem Siedepunkt des angewandten Lösungsmittels, vorzugsweise zwischen 5 und 100° durchgeführt.

Im alkalischen Medium treten oxidable Zwischenstufen auf, die sich durch eine Gelb- bis Violett-Färbung zu erkennen geben. Daher empfiehlt es sich, die Durchführung der Cyclisierungsreaktion im basischen Medium in einer Inertgasatmosphäre, z.B. unter Stickstoff vorzunehmen.

Die erfindungsgemäßen Verbindungen der Formel I bilden mit Basen salzartige Verbindungen. Daher wird die Aufarbeitung der Reaktionsansätze in der Regel so vorgenommen, daß man vor Isolierung der Reaktionsprodukte die der eingesetzten Base mindestens äquivalente Menge einer Säure, z.B. Salzsäure, Schwefelsäure oder Essigsäure, zusetzt. Man kann auch so vorgehen, daß man die in der Regel aus dem Reaktionsmedium ausfallenden Alkali- bzw. Erdalkalisalze durch Filtration oder Absaugen aus dem Reaktionsgemisch entfernt und nachträglich mit der mindestens äquivalenten Menge einer wäßrigen Säure behandelt.

Die Wirkstoffe können in bekannter Weise in die üblichen Formulierungen, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel überführt werden. Hierbei sollte die therapeutisch wirksame Verbindung jeweils in einer Konzentration von etwa 0,5 bis 90 Gew.-% der Gesamtmischung vorhanden sein, d.h. in Mengen, die ausreichend sind, um den angestrebten Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B.

im Falle der Benutzung von Wasser als Verdünnungmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle, (z.B. Erdnuß-/Sesamöl), Alkohole (z.B. Ethylalkohol, Glycerin), Glykole (z.B. Propylenglykol, Polyethylenglykol), N-Alkylpyrrolidone, feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-,Milch- und Traubenzucker). Emulgiermittel, wie nicht-ionogene und anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin,-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere cutan. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie

Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder gefärbten bzw. farbgebenden Stoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht bzw. der Art des Applikationsweges, aber auch aufgrund der Art und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle

der Applikation größerer Mengen kann es empfehlenswert sein, diese in mehrere Einzelgaben über den Tag zu verteilen. Die obigen Ausführungen gelten für die Applikation sowohl in der Human- als auch in der Tiermedizin in sinngemäß gleicher Weise.

Die folgenden Beispiele sollen die Erfindung näher erläutern.

Beispiel 1

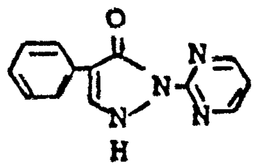

19,2 g (0,1 Mol) α-Hydroxymethylen-phenylessigsäure-ethylester und 11 g (0,1 Mol) 2-Hydrazino-pyrimidin werden in 150 ml Ethanol 3 Stunden unter Rückfluß gekocht. Das Gemisch wird auf Raumtemperatur abgekühlt und anschließend tropfenweise unter Rühren mit 9 g (0,1 Mol) konzentrierter Natronlauge versetzt. Man rührt 2 Stunden bei Raumtemperatur nach und kocht anschließend 2 Stunden unter Rückfluß. Das Gemisch wird mit konzentrierter Salzsäure neutralisiert und mit 1 ltr. Wasser verdünnt. Die abgeschiedenen Kristalle werden abgesaugt und an der Luft getrocknet. Ausbeute: 15 g (63 % der Theorie) 1-Pyrimidyl-(2)-4-phenyl-pyrazolin-5-on. F: 159 bis 160°C (aus Ethanol).

Beispiel 2

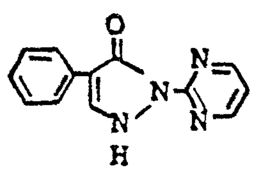

20,6 g (0,1 Mol) α-Methoxymethylen-phenylessigsäure-ethylester ($Kp_{0,2}$: 103-106°C) werden mit 11 g (0,1 Mol) 2-Hydrazino-pyrimidin in 100 ml Dioxan 24 Stunden unter

Rückfluß erhitzt. Das Lösungsmittel wird im Vakuum abdestilliert, der Rückstand in 50 ml Toluol verrührt. Dabei scheiden sich 3,2 g (13,5 % der Theorie) 1-Pyrimidyl-(2)-4-phenyl-pyrazolin-5-on ab, die abgesaugt und getrocknet werden. F: 159-160°C (aus Ethanol).

Beispiel 3

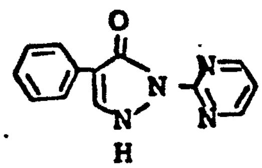

19,2 g (0,1 Mol) α-Hydroxymethylen-phenylessigsäureethylester werden in 100 ml Acetonitril gelöst und zunächst mit 11,2 g (0,1 Mol) Kalium-tert.-butylat, dann bei 20 bis 25°C portionsweise mit 19 g (0,1 Mol) p-Toluol-sulfochlorid versetzt. Man rührt 4 Stunden bei Raumtemperatur und läßt über Nacht stehen. Das abgeschiedene Salz wird abgesaugt, das Filtrat im Vakuum eingedampft. Der Rückstand wird in Toluol gelöst, die Lösung mit Wasser gewaschen, über $Na_2SO_4$ getrocknet und im Vakuum eingedampft. Ausbeute: 23 g (73 % der Theorie) α-(4-Methyl-phenyl-sulfonyloxymethylen)-phenylessigsäureethylester. Das Öl wird in 150 ml Ethanol gelöst und dann mit 7,7 g (0,07 Mol) 2-Hydrazino-pyrimidin versetzt. Das Gemisch wird 10 Stunden bei Raumtemperatur gerührt und dann im Verlaufe von 4 Stunden tropfenweise mit 13,5 g konzentrierter Natronlauge versetzt. Nach weiterem 5-stündigem Rühren bei Raumtemperatur wird verdünnte Salzsäure bis zum Neutralpunkt zugetropft und mit 750 ml Wasser verdünnt. Das sich abscheidende ölige Produkt wird in wenig Ether aufgenommen, wobei sich 3,6 g (22 % der Theorie) 1-Pyrimidyl-(2)-4-phenyl-pyrazolin-5-on abscheiden. F: 159 bis 160°C (aus Ethanol).

Beispiel 4

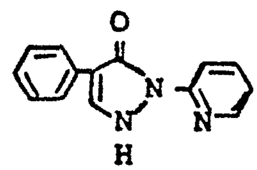

22,7 g (0,1 Mol) K-Salz des α-Hydroxymethylen-phenylessigsäureethylester und 18,2 g (0,1 Mol) 2-Hydrazinopyridin-dihydrochlorid werden in 200 ml Ethanol 30 Minuten bei Raumtemperatur verrührt, anschließend 5 Stunden unter Rückfluß gekocht. Nach dem Abkühlen auf Raumtemperatur werden unter gleichzeitigem Überleiten von Stickstoff portionsweise 19,1 g (1,7 Mol) Kalium-tert.-butylat eingetragen. Die Mischung wird 4 Stunden bei Raumtemperatur gerührt und über Nacht stehengelassen. Der gebildete Niederschlag wird abgesaugt, in 100 ml Wasser angeschlämmt und mit Essigsäure schwach angesäuert. Die Kristalle werden abgesaugt, mit Wasser gewaschen und an der Luft getrocknet. Man erhält 17,2 g (72,5 % der Theorie) 1-Pyridyl-(2)-4-phenyl-pyrazolin-5-on. F: 132 bis 133°C (Ethanol).

Beispiel 5

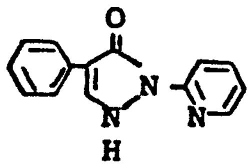

21,2 g (0,1 Mol) Phenylessigsäure-phenylester werden mit 11,9 g (0,1 Mol) Dimethylformamid-dimethylacetal 5 Stunden auf 100°C erhitzt. Dann werden bei dieser Temperatur im Wasserstrahlvakuum alle flüchtigen Bestandteile abdestilliert. Der im wesentlichen aus α-Dimethylaminomethylen-phenylessigsäure-phenylester bestehende Rückstand wird ohne weitere Reinigung in 100 ml Ethanol gelöst, die Lösung mit 18,2 g (0,1 Mol) 2-Hydrazinopyridin-dihydrochlorid versetzt und 5 Stunden unter Rückfluß gekocht. Nach dem Abkühlen scheiden sich 6,4 g (27 % der Theorie) des Reaktionsproduktes ab. Dieses wird abgesaugt und getrocknet. Die verbleibende ethanolische Lösung wird bei Raumtemperatur unter Stickstoff portionsweise mit 18,2 g (0,16 Mol) Kalium-tert.-butylat versetzt. Nach 5-stündigem Rühren bei Raumtemperatur wird der Niederschlag abgesaugt, in 100 ml Wasser angeschlämmt und mit Essigsäure angesäuert. Die Kristalle werden abgesaugt, mit Wasser gewaschen und an der Luft getrocknet. Dabei erhält man weitere 8,2 g des Reaktionsproduktes. Gesamtausbeute an 1-Pyridyl-(2)-4-phenyl-pyrazolin-5-on: 14,6 g (61,6 % der Theorie).

Beispiel 6

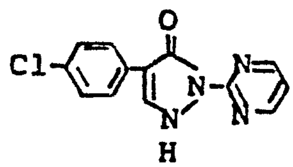

19,9 g (0,1 Mol) 4-Chlor-phenylessigsäure-ethylester werden mit 23,8 g (0,2 Mol) Dimethylformamid-dimethylacetal 5 Stunden auf 100°C erhitzt. Anschließend werden im Wasserstrahlvakuum bei dieser Temperatur alle flüchtigen Bestandteile abdestilliert. Der im wesentlichen aus α-Dimethylaminomethylen-4-chlor-phenylessigsäure-ethylester bestehende ölige Rückstand wird ohne weitere Reinigung in 140 ml Ethanol gelöst. Hierzu gibt man 11 g (0,1 Mol) 2-Hydrazino-pyrimidin und 10 ml konzentrierte Salzsäure. Das Gemisch wird 5 Stunden unter Rückfluß erhitzt, auf Raumtemperatur abgekühlt und nach Zugabe von 9 g konzentrierter Natronlauge 5 Stunden bei Raumtemperatur verrührt. Nach Neutralisation mit verdünnter Salzsäure wird mit 1 ltr. Wasser verdünnt. Die abgeschiedenen Kristalle werden abgesaugt, mit Wasser gewaschen und an der Luft getrocknet. Man erhält 7,2 g (26,4 % der Theorie) 1-Pyrimidyl-(2)-4-(4-chlor-phenyl)-pyrazolin-5-on. F: 190 bis 191°C (Methanol).

Analog wurden die Verbindungen der folgenden Beispiele hergestellt (Tabelle 1).

Tabelle 1

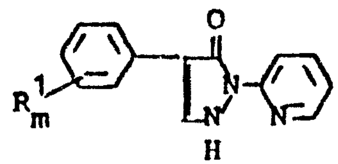

| Beisp.-Nr. | $R^1$ | m | Schmelzpunkt (°C) | Umkristallisieren aus |
|---|---|---|---|---|
| 7 | - | 0 | 131-133 | a |
| 8 | 2-F | 1 | 128-130 | g |
| 9 | 4-F | 1 | 137-139 | a |
| 1o | 2-Cl | 1 | 109-111 | a |
| 11 | 3-Cl | 1 | 146-147 | a |
| 12 | 4-Cl | 1 | 175-177 | b |
| 13 | 2-Br | 1 | 108 | b |
| 14 | 2-F, 6-Cl | 2 | 167-168 | b |
| 15 | $2,4-Cl_2$ | 2 | 168-169 | b |
| 16 | $3,4-Cl_2$ | 2 | 184-185 | b |
| 17 | $2-OCH_3$ | 1 | 110-112 | a |
| 18 | $4-OCH_3$ | 1 | 104-106 | a |
| 19 | $3-OC_6H_5$ | 1 | 148-149 | b |
| 2o | $3-OCF_3$ | 1 | 114-116 | a |

Tabelle 1 (Fortsetzung)

| Beisp.-Nr. | $R^1$ | m | Schmelzpunkt (°C) | Umkristallisieren aus |
|---|---|---|---|---|
| 21 | 4-$OCF_3$ | 1 | 120-122 | g |
| 22 | 4-$SCF_3$ | 1 | 186-188 | a |
| 23 | -O-$CF_2$-O- (3,4-Stellg.) | 2 | 179-181 | c |
| 24 | 2-$CH_3$ | 1 | 86- 88 | b |
| 25 | 3-$CH_3$ | 1 | 154-156 | a |
| 26 | 4-$CH_3$ | 1 | 132-133 | a |
| 27 | 3-$CF_3$ | 1 | 130-131 | a |
| 28 | 4-$CF_3$ | 1 | 171-173 | b |
| 29 | 2-Cl, 5-$CF_3$ | 2 | 116-118 | g |
| 30 | 3-$CF_3$, 4-Cl | 2 | 204-206 | b |
| 31 | 3,5-$(CF_3)_2$ | 2 | 174-176 | c |
| 32 | -CH=CH-CH=CH- (2,3-Stellg.) | 2 | 146 | a |
| 33 | 2-Cl, 5-$CF_3$ | 2 | 116-118 | g |
| 34 | 4-$OC_2H_5$ | 1 | 122 | a |
| 35 | 4-$OC_3H_7$ | 1 | 114-116 | a |
| 36 | 3,5-$(CH_3)_2$ | 2 | 150-152 | a |

Tabelle 1 (Fortsetzung)

| Beisp.-Nr. | $R^1$ | m | Schmelzpunkt. (°C) | Umkristall. aus |
|---|---|---|---|---|
| 37 | $4-COOCH_3$ | 1 | 163-165 | a |
| 38 | $3-COOC_2H_5$ | 1 | 109-111 | a |
| 39 | $4-NH-CO-CH_3$ | 1 | 203-205 | f |
| 40 | $4-SO_2N(CH_3)_2$ | 1 | 212-214 | b |
| 41 | 3-COOH | 1 | 245-247 | a |
| 42 | 4-COOH | 1 | > 270 | b |
| 43 | $4-SO_2-N=CH-N(CH_3)_2$ | 1 | 227-229 | f |
| 44 | $4-SO_2-N\begin{matrix} CH_3 \\ C_6H_5 \end{matrix}$ | 1 | 198-200 | b |
| 45 | $3-CO-NH-C(CH_3)_3$ | 1 | 172-174 | f |
| 46 | $4-SO_2-NH_2$ | 1 | > 260 | h + d |
| 47 | $4-CO-NH-C_6H_5$ | 1 | 242-244 | f |
| 48 | $3,4-O-CH_2-O-$ | 2 | 166-168 | f |

Tabelle 2

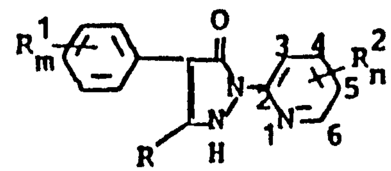

| Beisp.-Nr. | $R^1$ | m | R | $R^2$ | n | Schmelzpkt. (°C) | Umkristallisieren aus |
|---|---|---|---|---|---|---|---|
| 49 | - | 0 | $CH_3$ | - | 0 | 114-116 | a |
| 50 | - | 0 | $C_2H_5$ | - | 0 | 72- 73 | g |
| 51 | - | 0 | H | 6-Cl | 1 | 155-157 | b |
| 52 | 2-F, 6-Cl | 2 | H | 6-Cl | 1 | 220-221 | f |
| 53 | 3,4-$Cl_2$ | 2 | H | 6-Cl | 1 | 207 | f |
| 54 | 4-$CF_3$ | 1 | H | 6-Cl | 1 | 160-161 | b |
| 55 | - | 0 | H | 5-Cl | 1 | 209-211 | f |
| 56 | 2-Cl | 1 | H | 5-Cl | 1 | 195-197 | b |
| 57 | 3,4-$Cl_2$ | 2 | H | 5-Cl | 1 | 210-211 | b |
| 58 | 2-$OCH_3$ | 1 | H | 5-Cl | 1 | 176-177 | b |
| 59 | 2-$CH_3$ | 1 | H | 5-Cl | 1 | 188-190 | b |
| 60 | 3-$CH_3$ | 1 | H | 5-Cl | 1 | 156-158 | b |
| 61 | 4-$CH_3$ | 1 | H | 5-Cl | 1 | 207 | b |
| 62 | 4-$CF_3$ | 1 | H | 5-Cl | 1 | 218-220 | f |

Tabelle 2 (Fortsetzung)

| Beisp.-Nr. | $R^1$ | m | R | $R^2$ | n | Schmelzpkt. (°C) | Umkristall. aus |
|---|---|---|---|---|---|---|---|
| 63 | - | 0 | $CH_3$ | 5-Cl | 1 | 156-157 | g |
| 64 | - | 0 | H | 5-Cl | 1 | 164-166 | a |
| 65 | 2-Cl | 1 | H | 3,5-$Cl_2$ | 2 | 198-199 | a |
| 66 | 3,4-$Cl_2$ | 2 | H | 3,5-$Cl_2$ | 2 | 112-114 | f |
| 67 | 2-$CH_3$ | 1 | H | 3,5-$Cl_2$ | 2 | 164-166 | c |
| 68 | 2-$OCH_3$ | 1 | H | 5-Br | 1 | 184-186 | b |
| 69 | - | 0 | H | 5-$NO_2$ | 1 | 264 (Zers.) | f |
| 70 | 4-$CH_3$ | 1 | H | 5-$NO_2$ | 1 | 267-268 | h |
| 71 | 4-$CF_3$ | 1 | H | 5-$NO_2$ | 1 | 196-198 | i und d |
| 72 | - | 0 | H | 6-$CH_3$ | 1 | 110-112 | g |
| 73 | - | 0 | H | 4-$CH_3$ | 1 | 171-173 | b |
| 74 | - | 0 | H | 4,6-$(CH_3)_2$ | 2 | 147-149 | g |
| 75 | - | 0 | H | 4-Cl,6-$CH_3$ | 2 | 214-216 | a |
| 76 | 4-$CF_3$ | 1 | H | 4-Cl,6-$CH_3$ | 2 | 208-210 | a |

Tabelle 2 (Fortsetzung)

| Beisp.-Nr. | $R^1$ | m | R | $R^2$ | n | Schmelzpkt. (°C) | Umkristall. aus |
|---|---|---|---|---|---|---|---|
| 77 | - | 0 | H | $5-CF_3$ | 1 | 172-174 | a |
| 78 | - | 0 | H | 5-CN | 1 | 243-245 | b |
| 79 | - | 0 | H | $3-CN,6-CH_3$ | 2 | 300 | a |
| 80 | 2-Cl | 1 | H | $5-CO-NH_2$ | 1 | 270 | h |
| 81 | - | 0 | H | -CH=CH-CH=CH-; $4-CH_3$ (5,6-Stellg.) | 3 | 180-182 | b |
| 82 | $3,4-Cl_2$ | 2 | H | -CH=CH-CH=CH-; $4-CH_3$ (5,6-Stellg.) | 3 | 228-230 | f |
| 83 | $4-CH_3$ | 1 | H | -CH=CH-CH=CH-; $4-CH_3$ (5,6-Stellg.) | 3 | 196 | b |
| 84 | $4-CF_3$ | 1 | H | -CH=CH-CH=CH-; $4-CH_3$ (5,6-Stellg.) | 3 | 252-254 | b |

Tabelle 2 (Fortsetzung)

| Beispiel-Nr. | $R_1$ | m | R | $R_2$ | n | F(°C) | Umkristall. aus |
|---|---|---|---|---|---|---|---|
| 85 | - | 0 | $C_3H_7$ | - | 0 | 76-78 | g |
| 86 | - | 0 | H | 6-$CH_3$ | 1 | 110-112 | g |
| 87 | 4-$OCH_3$ | 1 | $CH_3$ | - | 0 | 126-128 | g |
| 88 | 2-F | 1 | $CH_3$ | - | 0 | 98-100 | g |
| 89 | 4-$CH_3$ | 1 | $CH_3$ | - | 0 | 114-115 | g |
| 90 | 4-$OCH_3$ | 1 | $CH_3$ | 5-Cl | 1 | 180-181 | c |
| 91 | 4-$OCH_3$ | 1 | H | 5-Cl | 1 | 167-169 | c |
| 92 | 3,4 $Cl_2$ | 2 | $CH_3$ | - | 0 | 128-130 | g |
| 93 | 4-$OCH_3$ | 1 | H | 4-$CH_3$ | 1 | 146-148 | g |
| 94 | - | 0 | $CH_3$ | 4-$CH_3$ | 1 | 144-145 | g |
| 95 | 3-$CH_3$ | 1 | $CH_3$ | 5-Cl | 1 | 174-176 | g |
| 96 | 4-$CH_3$ | 1 | $CH_3$ | 5-Cl | 1 | 196-198 | g |
| 97 | - | 0 | $CH_3$ | 5-$CH_3$ | 1 | 121-123 | g |
| 98 | - | 0 | H | 5-$CH_3$ | 1 | 170-172 | c |
| 99 | 4-$OCH_3$ | 1 | H | 5-$CH_3$ | 1 | 164-166 | b |

Tabelle 3

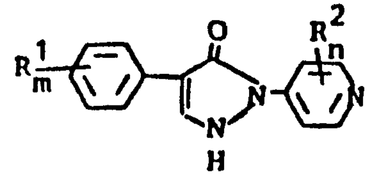

| Beisp.-Nr. | $R^1$ | m | $R^2$ | n | Schmelzpunkt (°C) | Umkristall. aus |
|---|---|---|---|---|---|---|
| 100 | - | 0 | - | 0 | 242-246 | i |
| 100 | - | 0 | 2,6-$(CH_3)_2$ | 2 | 254 | i |
| 102 | 4-$CF_3$ | 1 | 2,6-$(CH_3)_2$ | 2 | 260-264 | b |

Tabelle 4

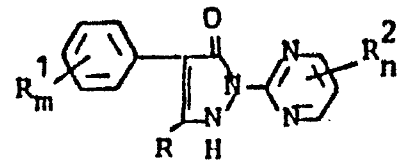

| Beisp.-Nr. | $R^1$ | m | R | $R^2$ | n | Schmelzpunkt (°C) | Umkristall. aus |
|---|---|---|---|---|---|---|---|
| 103 | - | 0 | H | - | 0 | 158-160 | a |
| 104 | 2-F | 1 | H | - | 0 | 168-170 | a |
| 105 | 4-F | 1 | H | - | 0 | 210-212 | a |
| 106 | 2-Cl | 1 | H | - | 0 | 140-150 | a |
| 107 | 4-Cl | 1 | H | - | 0 | 190-191 | d |
| 108 | 2-Br | 1 | H | - | 0 | 137-138 | a |
| 109 | 2-F, 6-Cl | 2 | H | - | 0 | 178 | b |
| 110 | $2,4-Cl_2$ | 2 | H | - | 0 | 243-244 | b |
| 111 | $3,4-Cl_2$ | 2 | H | - | 0 | 246-247 | f |
| 112 | $2-OCH_3$ | 1 | H | - | 0 | 130 | a |
| 113 | $4-OCH_3$ | 1 | H | - | 0 | 166-168 | c |
| 114 | $3-OCF_3$ | 1 | H | - | 0 | 162-164 | c |

Tabelle 4 (Fortsetzung)

| Beisp.-Nr. | $R^1$ | m | R | $R^2$ | n | Schmelzpunkt (°C) | Umkristall. aus |
|---|---|---|---|---|---|---|---|
| 115 | 4-$OCF_3$ | 1 | H | - | 0 | 170-172 | a |
| 116 | 4-$SCF_3$ | 1 | H | - | 0 | 202-204 | b |
| 117 | -O-$CF_2$-O- (3,4-Stellg.) | 2 | H | - | 0 | 230-231 | f |
| 118 | 2-$CH_3$ | 1 | H | - | 0 | 133-134 | b |
| 119 | 3-$CH_3$ | 1 | H | - | 0 | 156-158 | a |
| 120 | 4-$CH_3$ | 1 | H | - | 0 | 168-170 | a |
| 121 | 3-$CF_3$ | 1 | H | - | 0 | 180-181 | b |
| 122 | 4-$CF_3$ | 1 | H | - | 0 | 226 | b |
| 123 | 2-$CF_3$, 4-Cl | 2 | H | - | 0 | 150-152 | a |
| 124 | 2-Cl; 5-$CF_3$ | 2 | H | - | 0 | 182-184 | c |
| 125 | 3-$CF_3$; 4-Cl | 2 | H | - | 0 | 188-189 | a |
| 126 | 3,5-$(CF_3)_2$ | 2 | H | - | 0 | 188-189 | a |
| 127 | -CH=CH-CH=CH- (2,3-Stellg.) | 2 | H | - | 0 | 146-148 | b |

Tabelle 4 (Fortsetzung)

| Beisp.-Nr. | $R^1$ | m | R | $R^2$ | n | Schmelzpunkt (°C) | Umkristall. aus |
|---|---|---|---|---|---|---|---|
| 128 | - | 0 | $CH_3$ | - | 0 | 202-204 | b |
| 129 | - | 0 | H | 5-F | 1 | 245-246 | f |
| 130 | - | 0 | H | 5-Cl | 1 | 267-268 | i |
| 131 | 2-Cl | 1 | H | 5-Cl | 1 | 216-218 | b |
| 132 | 3,4-$Cl_2$ | 2 | H | 5-Cl | 1 | 242-244 | f |
| 133 | 2-$CH_3$ | 1 | H | 5-Cl | 1 | 204 | b |
| 134 | 4-$CH_3$ | 1 | H | 5-Cl | 1 | 264 | b |
| 135 | 4-$CF_3$ | 1 | H | 5-Cl | 1 | 268-270 | f |
| 136 | - | 0 | H | 6-$CH_3$ | 1 | 204-206 | a |
| 137 | - | 0 | H | 4,6-$(CH_3)_2$ | 2 | 256 | c |
| 138 | 4-F | 1 | H | 4,6-$(CH_3)_2$ | 2 | 275 | b |
| 139 | 4-Cl | 1 | H | 4,6-$(CH_3)_2$ | 2 | $>$ 270 | h |
| 140 | - | 0 | $CH_3$ | 4,6-$(CH_3)_2$ | 2 | 181-182 | c |

Tabelle 5

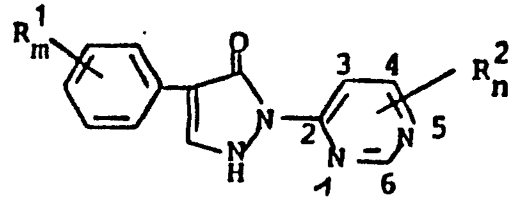

| Beisp.-Nr. | $R^1$ | m | $R^2$ | n | Schmelzpunkt (°C) | Umkristall. aus |
|---|---|---|---|---|---|---|
| 141 | - | 0 | $4,6-(CH_3)_2$ | 2 | 192 | b |
| 142 | $4-CF_3$ | 1 | $4,6-(CH_3)_2$ | 2 | 196-198 | i |
| 143 | - | 0 | $4-Cl, 6-CH_3$ | 2 | 162-164 | g |
| 144 | - | 0 | $3-OCH_3,6-t-C_4H_9$ | 2 | 175 | b |
| 145 | $3,4-Cl_2$ | 2 | $3-OCH_3,6-t-C_4H_9$ | 2 | 196-197 | b |
| 146 | $4-CF_3$ | 1 | $3-OCH_3,6-t-C_4H_9$ | 2 | 200 | b |
| 147 | - | 0 | $4-CH_3,6-SCH_3$ | 2 | 180-182 | c |
| 148 | $4-CF_3$ | 1 | $4-CH_3,6-SCH_3$ | 2 | 174-176 | g |

Lösungsmittel

a Ethanol
b Butanol
c Toluol
d Methanol
e Essigester
f Dioxan
g Waschbenzin
h Dimethylformamid
i Glykolmonomethylether

Die biologische Wirkung der erfindungsgemäßen Verbindungen wurde durch folgende Experimente nachgewiesen:

A) Lipoxygenasehemmung/Cyclooxygenasehemmung/Prostacyclinstimulation

1. Lipoxygenase- und Cyclooxygenaseaktivität in Homogenaten von RBL-1-Zellen

Basophile Leukämie-Zellen von Ratten (RBL-1) synthetisieren aus zugeführter $^{14}C$-Arachidonsäure sowohl Lipoxygenase- als auch Cyclooxygenase-Produkte. Damit ergibt sich die Möglichkeit, die relative Potenz und Selektivität von Prüfsubstanzen zu erfassen.
Die entstehenden Produkte wurden dünnschichtchromatographisch getrennt und im Scanner quantifiziert.

2. Lipoxygenase-aktivität in menschlichen PMN-Leukozyten

Selektiv wird die für die Biosynthese der Leukotriene $B_4$, $C_4$ und $D_4$ verantwortliche 5-Lipoxygenase bei niedrigen Konzentrationen gehemmt. Die polymorphkernigen Leukozyten des Menschen metabolisieren die Arachidonsäure zu 5-Hydroxy-5,8,11,14-eikosatetraensäure (5-HETE) und Leukotrien $B_4$ (5S, 12R-Dihydroxy-6 cis, 8,10-trans-14 cis-eikosatetraensäure). Die Hemmung der Freisetzung von 5-HETE und Leukotrien $B_4$ aus den Leukozyten stellt ein Maß für den lipoxygenasehemmenden Effekt der erfindungsgemäßen Verbindungen dar.

Der Test mit den Humanleukozyten wurde nach Borgeat und Samuelsson (J. Biol. Chem. 254, 2643, 1979 und Proc. Natl. Acad. Sci. USA, 76, 2148, 1979) durchgeführt.

Die im vorliegenden Beispiel verwendeten Human PMN-Leukozyten ( > 95 %) wurden aus heparinisiertem Vollblut durch eine Dextran-Sedimentation und anschließende Dichtegradiententrennung (Ficoll-Paque) gewonnen (vgl. A. Boyum, Scand. J. Immunol., 5, Suppl. 5, 9, 1976).

2 x $10^7$ Zellen/ml wurden in $Ca^{2+}$-haltigem Dulbecco-Phosphat-Puffer suspendiert und in Anwesenheit bzw. Abwesenheit von Lipoxygenasehemmer mit dem Calcium-Ionophor A 23187 stimuliert. Nach 15 Minuten wurden die Lipoxygenaseprodukte aus dem angesäuerten Inkubationsmedium extrahiert und mittels HPLC getrennt.

3. $^3H$-Arachidonsäuremetabolismus in menschlichen Thrombozyten (12-Lipoxygenaseaktivität und Cyclooxygenaseaktivität)

Der Nachweis der lipoxygenasehemmenden und cyclooxygenasehemmenden Eigenschaften der erfindungsgemäßen Verbindungen erfolgte analog der Methode von Bailey et al., Journal of Biol. Chemistry 255, 5996, 1980 und nach Blackwell und Flower, Prostaglandins 16, 417, 1978. Bei dieser Testmethode wird der Metabolismus radioaktiv markierter Arachidonsäure an gewaschenen Humanthrombozyten herangezogen. Bei diesem in vitro Test werden die radioaktiv

markierten Metaboliten aus dem Reaktionsansatz extrahiert und dünnschichtchromatographisch getrennt. Das Autoradiogramm wird am Dünnschichtscanner ausgewertet. Bei diesen Testbedingungen werden die markierten Metaboliten von der nicht umgesetzten Arachidonsäure getrennt und sind anschließend quantitativ auswertbar. Die Verteilung der Radioaktivität auf die während der Metabolisierung gebildeten Cyclooxygenaseprodukte Thromboxan $A_2$ (bestimmt als $TXB_2$) und 12-Hydroxy-5,8,10-heptadekatriensäure (HHT) bzw. das Lipoxygenaseprodukt 12-Hydroxy-5,8,11,14-eikosatetraensäure (12-HETE) unter dem Einfluß der Inhibitoren stellt ein Maß für die Hemmung der Enzyme dar.

4. Prostacyclinstimulation

Außerdem stimulieren die erfindungsgemäß zu verwendenden Verbindungen spezifisch die Systhese von Prostacyclin ($PGI_2$). $PGI_2$ wirkt im Gegensatz zu dem vasokonstriktorischen und thrombozytenaggregationsauslösenden Thromboxan gefäßdilatierend und thrombozytenaggregationshemmend.

a) Stimulation in Vollblut.
In Vollblut läßt sich durch Kollagen die Bildung von $PGI_2$ induzieren. Die in Thrombozyten gebildeten Endoperoxide

werden wahrscheinlich durch Leukozyten-Lipoxygenase in $PGI_2$ umgewandelt. Das stabile Endprodukt der $PGI_2$-Anwendung, 6-Keto-$PGF_{1\alpha}$, wird radioimmunologisch bestimmt.

b) Stimulation in Mikrosomen in vitro.
Die spezifische $PGI_2$-stimulierende Wirkung wurde in vitro in einem Gemisch aus Mikrosomen von Schafssamenblasen (RSVM) und Rinderaorten (BAM) gezeigt (vgl. F. Cottee et al., Prostaglandins, 14, 413, 1977). $^3$H-Arachidonsäure wurde in Gegenwart der erfindungsgemäßen Verbindungen mit einem Gemisch aus RSVM und BAM 10 Minuten bei 25°C inkubiert. Die Reaktion wurde durch Ansäuern auf pH 3,5 gestoppt. Die Fettsäuremetaboliten wurden mit Essigester extrahiert. Der Essigester wurde unter $N_2$ abgedampft und der Rückstand in $CH_3OH/CHCl_3$ (1:1) aufgenommen und auf DC-Plastikfolien aufgetragen. Die Trennung erfolgte mit einem Fließmittelgemisch Ethylenacetat/Eisessig/Isooctan/$H_2O$ (110:20:50:10; organische Phase) (P. Needleman et al., The Journal of Clinical Investigation 1978, 61, 839-849). Die Verteilung der Radioaktivität wurde mittels eines Radioscanners gemessen.

## B) Allergie/Asthma und andere Erkrankungen des Respirationstraktes

### 1. Freisetzung von Mediatoren aus menschlichen Lungen

In Anlehnung an die pathophysiologische Situation (Typ I - Überempfindlichkeitsreaktion) werden Lungen mit Serum passiv sensibilisiert. Zugabe eines entsprechenden, spezifischen Allergens führt zur Freisetzung von Mediatoren der Anaphylaxie wie Histamin, SRS-A, Thromboxan und Prostaglandinen.
Da beim Menschen die Lunge das wichtigste Zielorgan für allergische Reaktionen ist, stellt dieser Test ein relevantes Modell zur Prüfung antiasthmatischer Substanzen dar.

### 2. Passive peritoneale Anaphylaxie (Ratte)

Mit dieser in vivo-Methode wird die Wirkung von Substanzen auf die Freisetzung von Mediatoren, insbesondere die Freisetzung von Leukotrienen, erfaßt. Die passive Sensibilisierung von peritonealen Mastzellen der Ratte mit Anti-Ovalbumin Meerschweinchenserum, führt nach intraperitonealer Injektion des Antigens zur Freisetzung von SRS-A (Smith et al., Int. Arch. Allergy appl. Immunol. 62, 195, 1980).
Histamin wird hierbei nicht freigesetzt, so daß die lipoxygenasehemmende Wirkung von Prüfsubstanzen selektiv untersucht werden kann.

C) Entzündliche Erkrankungen

Die entzündungshemmende Wirkung der Testsubstanzen nach oraler Applikation wurde mit Hilfe des durch Carrageenan induzierten Ödems der Rattenpfote ermittelt.

Die Versuche wurden mit männlichen Ratten, Stamm: Bor:WISW (SPF-Cpb); Gewicht 150 bis 220 g, durchgeführt. Eine Stunde vor Auslösung des Ödems (0,1 ml Carrageenan-Suspension/Tier, subplantar injiziert) wurde den Tieren die zu testende Substanz in Traganth suspendiert mit Hilfe einer Schlundsonde appliziert; zur Kontrolle wurde eine zweite Tiergruppe mit Traganth ohne Substanz behandelt.

Die Pfotenvolumina wurden nach der Methode von F. Kemper und G. Ameln Z. exp. Med. 131, 407 (1959) gemessen, wobei die Differenz des Pfotenvolumens 5 Stunden nach der Ödemprovokation vom normalen Pfotenvolumen das Ödemvolumen ergibt.

Zunächst wurde beispielhaft die $DE_{50}$ der Ödemhemmung für die Verbindung Beispiel 4 (7) nach oraler Applikation bestimmt. Aus den ermittelten Einzelwerten konnte eine $DE_{50}$ von 9,1 mg/kg p.o. errechnet werden. In weiterführenden Untersuchungen wurden die folgenden, in Tabelle 5 zusammengefaßten Pyrazolinone untersucht:

D) Protektion gegenüber einer durch Arachidonsäure induzierten Thromboembolie

In diesem Modell wirken Substanzen, die mit der Prostaglandin- und Thromboxanbiosynthese interferieren. Kaninchen werden 1,5 mg/kg Na-arachidonat in eine marginale Ohrvene injiziert. Die Tiere sterben innerhalb von Minuten, vermutlich aufgrund vasospastischer Cyclooxygenase- und Lipoxygenaseprodukte sowie embolisierender Plättchenthromben.

Literatur:
Silver, M.J. et al., Science 183, 1085, 1974
Seuter, F., Busse, W.D., Agents and Actions, Suppl. 4, 175, 1979.

E) Thrombozytenaggregationshemmung

Thrombozyten und deren Adhäsion - sowie Aggregationsfähigkeit - sind, besonders im arteriellen Schenkel des Gefäßsystems, ein wesentlicher pathogenetischer Faktor bei der Entstehung von Thrombosen.

Für die in vitro Versuche wurde Blut von gesunden Probanden beiderlei Geschlechts verwendet. Als Antikoagulans wurde ein Teil 3,0 %ige wäßrige Natriumzitratlösung 9 Teilen Blut zugemischt. Mittels Zentrifugation erhält man aus diesem Blut plättchenreiches Zitratplasma (PRP) (Literatur: Jürgens/Beller, Klinische Methoden der Blutgerinnungsanalyse; Thieme Verlag, Stuttgart 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (Literatur: Born, B.V.R., J. Physiol. (London), 162, 67, 1962) im Aggregometer bei 37°C bestimmt (Literatur: Therapeutische Berichte 47, 80-86, 1975). Hierzu wurde die vorinkubierte Probe mit 0,1 ml Kollagen, einem aggregationsauslösenden Agens, versetzt.

Die Veränderung der optischen Dichte in der Probe des PRP wurde während einer Zeitdauer von 6 Minuten aufgezeichnet und der Ausschlag nach 6 Minuten bestimmt. Hieraus wird die prozentuale Hemmung gegenüber der Kontrolle errechnet.

## Ergebnisse

A 1) Lipoxygenase- und Cyclooxygenaseaktivität in Homogenaten von RBL-1-Zellen (Tabelle 2)

Wie die Tabelle 2 zeigt, sind viele Verbindungen bei $5 \times 10^{-7}$ g/ml noch mit > 50 % Hemmung wirksam. Darüber hinaus ist die selektive Wirkung auf die Lipoxygenase klar erkennbar, die Cyclooxygenase wird bei den angegebenen Konzentrationen nicht oder nur geringfügig beeinflußt.

A 2) **Lipoxygenaseaktivität in menschlichen PMN-Leukocyten (Tabelle 3)**

Wie die Tabelle 3 zeigt wird bei $1 \times 10^{-5}$ g/ml eine noch fast vollständige Hemmung erreicht. Das therapeutisch bereits eingesetzte Benoxaprofen ist schwächer wirksam ($IC_{50} \sim 1\text{-}5 \times 10^{-5}$ g/ml).

A3) **Lipoxygenase- und Cyclooxygenase-aktivität in Thrombozyten (Tabelle 4)**

Parallel zur Hemmung der 5-Lipoxygenase in Leukozyten wird auch die 12-Lipoxygenase in Thrombozyten gehemmt. Die Beeinflussung der Cyclooxygenase in Thrombozyten ist als Hinweis auf eine antithrombotische Wirkung der Substanzen zu werten.

A4) Prostacyclinstimulation

Die erfindungsgemäßen Verbindungen stimulieren die $PGI_2$-Synthese.

B2) Passive peritoneale Anaphylaxie (Ratte) (Tabelle 5)

Wie die Tabelle zeigt, wird die Bildung von Leukotrienen nach oraler Gabe der Substanzen nahezu vollständig inkubiert.

C) Entzündungshemmung (Tabelle 6)

Die Hemmung einer entzündlichen Reaktion im Carrageenan-Ödem ist in der Tabelle 6 angegeben.

D) Thromboembolie

Die erfindungsgemäßen Verbindungen verhindern nach oraler Gabe eine durch Arachidonsäure ausgelöste Thromboembolie.

E) Thrombozytenaggregationshemmung

Die erfindungsgemäßen Verbindungen hemmen eine durch Kollagen ausgelöste Aggregation von Thrombozyten, was eine potentielle antithrombotische Wirkung anzeigt.

Tabelle 2 Wirkung in Homogenaten von RBL-1-Zellen auf die Lipoxygenase (LO) und Cyclooxygenase (CO)

| Beispiel | Konz. LO (µg/ml) | Effekt (%) | Konz. CO (µg/ml) | Effekt (%) |
|---|---|---|---|---|
| 7 | 0,5-1 | -66 | 0,5-1 | +28 |
| 16 | 0,5-1 | -76 | 0,5-1 | -19 |
| 58 | 0,5-1 | -66 | 0,5-1 | -17 |
| 24 | 0,5-1 | -34 | 0,5-1 | -2 |
| 8 | 0,5-1 | -73 | 0,5-1 | +3 |
| 28 | 0,5-1 | -58 | 0,5-1 | +5 |
| 103 | 0,5-1 | -42 | 0,5-1 | ±0 |
| 113 | 0,5-1 | -71 | 0,5-1 | -11 |
| 104 | 0,5-1 | -46 | 0,5-1 | +23 |

Tabelle 3 Hemmung der $LTB_4$-Synthese in Human-PMN's

| Beispiel | Hemmung bei 10 µg/ml (%) | wirksame Grenzkonzentration (µg/ml) |
|---|---|---|
| 16 | 85 | 1-10 |
| 7 | 92 | 1-10 |
| 8 | 71 | 1-10 |
| 63 | 100 | 1-10 |

Tabelle 4 Arachidonsäuremetabolismus in Human-Thrombozyten

| Substanz | Hemmung (Grenzkonzentration in µg/ml) 12-Lipoxygenase | Cyclooxygenase |
|---|---|---|
| 16 | 1-10 | 1-10 |
| 7 | < 0,1 | < 0,1 |
| 8 | 1-10 | 1-10 |
| 63 | 1-10 | 1-10 |

Tabelle 5:

Passive peritoneale Anaphylaxie (Ratte)

| Beispiel: | %-Hemmung nach Gabe von 100 mg/kg p.o. |
|---|---|
| 7 | 65 |
| Bay Q8921 Nr. 25 | 64 |
| Bay R1152 Nr. 27 | 47 |

Tabelle 6 Antiphlogistische Wirkung verschiedener Pyrazolinone
Applikationsart: p.o.
Dosis: 2,5 mg/kg

| Beispiel | % Hemmung des Carrageenan-Ödems |
|---|---|
| 28 | 21 % |
| 7 | 44 % |
| 130 | 38 % |
| 49 | 16 % |
| 18 | 22 % |
| 135 | 46 % |
| 69 | 34 % |

Patentansprüche

1. 1-Heteroaryl-4-aryl-pyrazolin-5-one der allgemeinen Formel

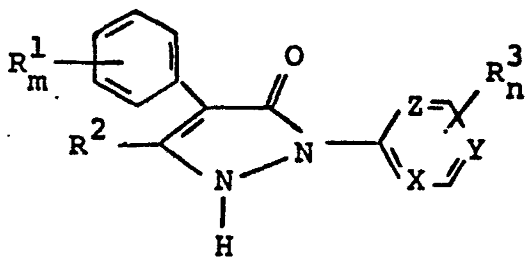

in der

$R^1$ Wasserstoff, Halogen, Hydroxy, gegebenenfalls substituiertes Niederalkoxy, gegebenengalls substituiertes Phenoxy, gegebenenfalls substituiertes Niederalkylmercapto, gegebenenfalls substituiertes Niederalkylsulfonyl, gegebenenfalls durch Fluor substituiertes Niederalkyl, einen ankondensierten carbocyclischen oder heterocyclischen Rest, Carboxyl, Niederalkoxycarbonyl oder eine der Gruppen

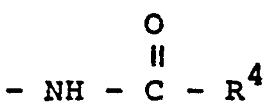 , 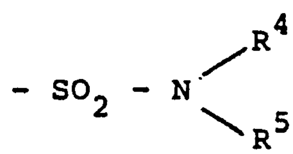 ,

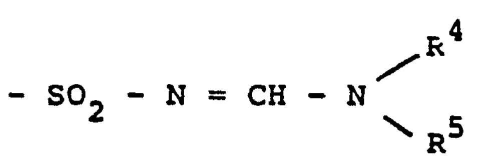 , 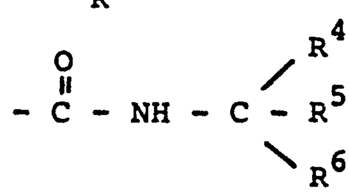

oder

$$-\overset{O}{\overset{\|}{C}}-NH-R^4$$

bedeutet
wobei
$R^4$, $R^5$ und $R^6$ gleich oder verschieden sind und Wasserstoff, Niederalkyl oder Phenyl bedeuten,

m eine ganze Zahl von 1 bis 5 bedeutet,

$R^2$ Wasserstoff oder Niederalkyl bedeutet,

X, Y und Z ein N-Atom oder ein Ringglied $=\overset{R^3}{\overset{|}{C}}-$ bedeutet, wobei wenigstens eines der X, Y oder Z ein N-Atom bedeutet,

$R^3$ Wasserstoff, Halogen, Niederalkoxy, Niederalkylmercapto, Niederalkyl, Niederhalogenalkyl, Nitro, Cyan, Carbonsäureamid oder einen ankondensierten Phenylenrest bedeutet, und

n eine ganze Zahl von 1 bis 4 bedeutet

zur Verwendung als Arzneimittel.

2. 1-Heteroaryl-4-aryl-pyrazolin-5-one nach Anspruch 1 zur Verwendung als Lipoxygenasehemmer.

3. 1-Heteroaryl-4-aryl-pyrazolin-5-one nach den Ansprüchen 1 und 2 zur Verwendung bei der Behandlung von Erkrankungen der Atemwege wie Allergien/Asthma, Bronchitis, Emphysem, Schocklunge, pulmonaler Hypertonie, Entzündungen/Rheuma, Arthrosen und Ödemen, Thrombosen und Thromboembolien, Ischämien (periphere, kardiale, cerebrale Durchblutungsstörungen), Herz- und Hirninfarkten, Herzrhythmusstörungen, Angina pectoris, Arteriosklerose, bei Gewebetransplantationen, Dermatosen Metastasen und zur Cytoprotektion im Gastrointestinaltrakt.

4. Stoffgemische, enthaltend 1-Heteroaryl-4-aryl-pyrazolin-5-one der allgemeinen Formel I, wobei diese teilweise oder ausschließlich in der tautomeren Form vorliegen können,

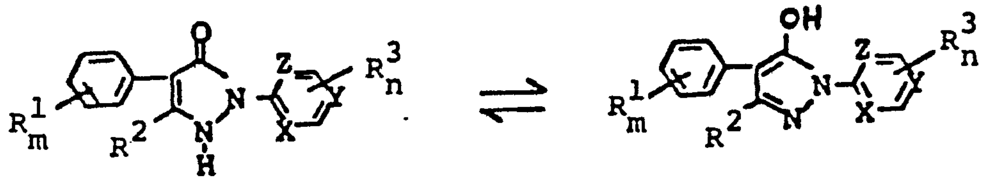

in der

$R^1$ Wasserstoff, Halogen, Hydroxy, gegebenenfalls substituiertes Niederalkoxy, gegebenengalls substituiertes Phenoxy, gegebenenfalls substituiertes Niederalkylmercapto, gegebenenfalls substituiertes Niederalkylsulfonyl, gegebenenfalls durch Fluor substituiertes Niederalkyl, einen ankondensierten carbocyclischen oder heterocyclischen Rest, Carboxyl, Niederalkoxycarbonyl oder eine der Gruppen

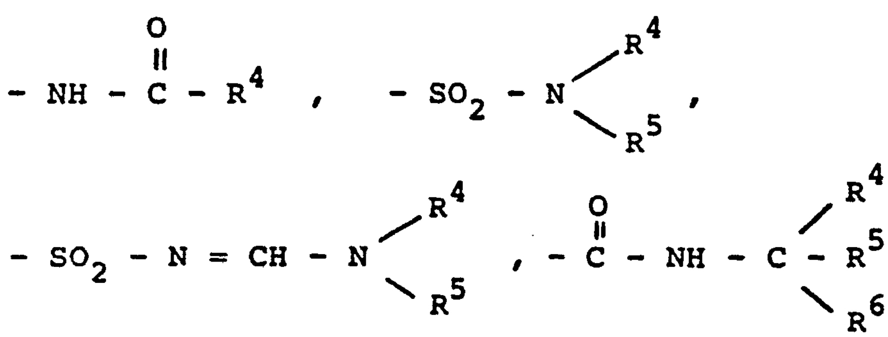

oder

$$-\overset{O}{\overset{\|}{C}}-NH-R^4$$

bedeutet
wobei

$R^4$, $R^5$ und $R^6$ gleich oder verschieden sind und Wasserstoff, Niederalkyl oder Phenyl bedeuten,

m eine ganze Zahl von 1 bis 5 bedeutet,

$R^2$ Wasserstoff oder Niederalkyl bedeutet,

X, Y und Z ein N-Atom oder ein Ringglied $=\overset{R^3}{\overset{|}{C}}-$ bedeutet, wobei wenigstens einer der X, Y oder Z ein N-Atom bedeutet,

$R^3$ Wasserstoff, Halogen, Niederalkoxy, Niederalkylmercapto, Niederalkyl, Niederhalogenalkyl, Nitro, Cyan, Carbonsäureamid oder einen ankondensierten Phenyllenrest bedeutet, und

n eine ganze Zahl von 1 bis 4 bedeutet,

zur Verwendung als Arzneimittel.

5. Stoffgemische nach Anspruch 4 zur Verwendung als Lipoxygenasehemmer.

6. Stoffgemische nach den Ansprüchen 4 und 5 zur Verwendung bei der Behandlung von Erkrankungen der Atemwege wie Allergien/Asthma, Bronchitis, Emphysem, Schocklunge, pulmonaler Hypertonie, Entzündungen/Rheuma, Arthrosen und Ödemen, Trombosen und Tromboembolien, Ischämien (periphere,kardiale, cerebrale Durchblutungsstörungen), Herz- und Hirninfarkten, Herzrythmusstörungen, Angina pectoris, Arteriosklerose, bei Gewebetransplantationen, Dermatosen, Metastasen und zur Cytoprotektion im Gastrointestinaltrakt.

7. Verwendung von 1-Hetero-4-aryl-pyrazolin-5-onen der allgemeinen Formeln, wobei diese teilweise oder ausschließlich in der tautomeren Form vorliegen können,

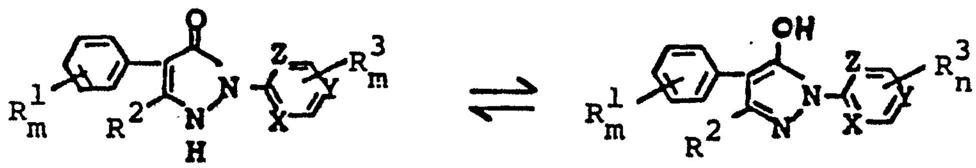

in der

$R^1$ Wasserstoff, Halogen, Hydroxy, gegebenenfalls substituiertes Niederalkoxy, gegebenenfalls substituiertes Phenoxy, gegebenenfalls substituiertes Niederalkylmercapto, gegebenenfalls substituiertes Niederalkylsulfonyl, gegebenenfalls durch Fluor substituiertes Niederalkyl, einen ankondensierten carbocyclischen oder heterocyclischen Rest, Carboxyl, Niederalkoxycarbonyl oder eine der Gruppen

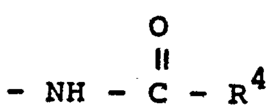 , 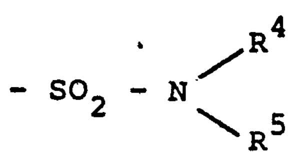 ,

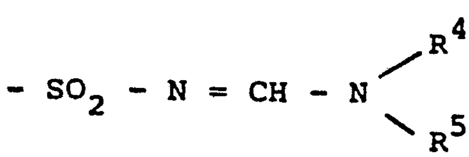 , 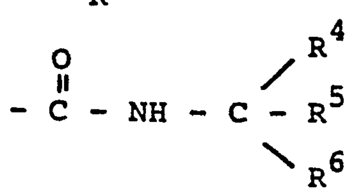

oder

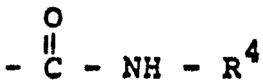

bedeutet
wobei

$R^4$, $R^5$ und $R^6$ gleich oder verschieden sind und Wasserstoff, Niederalkyl oder Phenyl bedeuten,

m eine ganze Zahl von 1 bis 5 bedeutet,

$R^2$ Wasserstoff oder Niederalkyl bedeutet,

X, Y und Z ein N-Atom oder ein Ringglied 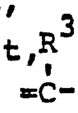 bedeuten, wobei wenigstens einer der X, Y oder Z ein N-Atom bedeutet,

$R^3$ Wasserstoff, Halogen, Niederalkoxy, Niederalkylmercapto, Niederalkyl, Halogen-niederalkyl, Nitro, Cyan, Carbonsäureamid oder einen ankondensierten Phenyllenrest bedeutet, und

n eine ganze Zahl von 1 bis 4 bedeutet,

zur Herstellung von Arzneimitteln.

8. Verwendung von 1-Hetero-4-arylpyrazolin-5-onen nach Anspruch 7 für die Herstellung von Arzneimitteln zur Lipoxygenasehemmung.

9. Verwendung von 1-Hetero-4-arylpyrazolin-5-onen nach den Ansprüchen 7 und 8 für die Herstellung von Arzneimitteln zur Bekämpfung entzündlicher und allergischer Prozesse im menschlichen oder tierischen Körper.

10. Verfahren zur Herstellung von Arzneimitteln, dadurch gekennzeichnet, daß man 1-Hetero-4-arylpyrazolin-5-onen, die teilweise oder ausschließlich in einer der tautomeren Formen vorliegen können, der allgemeinen Formeln

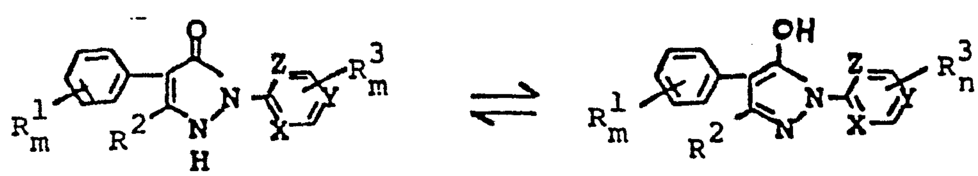

in denen

$R^1$ Wasserstoff, Halogen, Hydroxy, gegebenenfalls substituiertes Niederalkoxy, gegebenengalls substituiertes Phenoxy, gegebenenfalls substituiertes Niederalkylmercapto, gegebenenfalls substituiertes Niederalkylsulfonyl, gegebenenfalls durch Fluor substituiertes Niederalkyl, einen ankondensierten carbocyclischen oder heterocyclischen Rest, Carboxyl, Niederalkoxycarbonyl oder eine der Gruppen

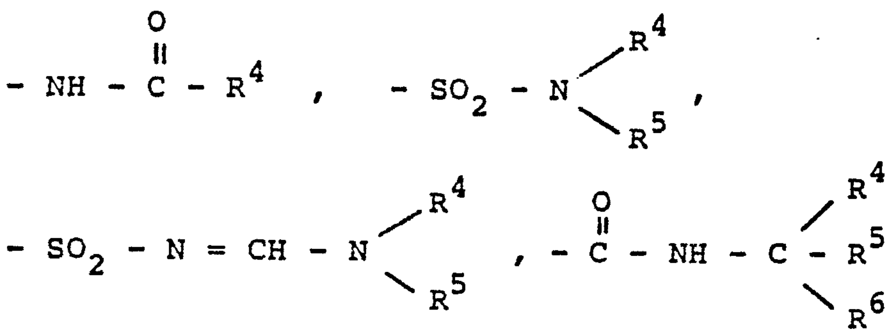

oder

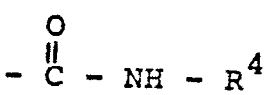

bedeutet

wobei

$R^4$, $R^5$ und $R^6$ gleich oder verschieden sind und Wasserstoff, Niederalkyl oder Phenyl bedeuten,

m eine ganze Zahl von 1 bis 5 bedeutet,

$R^2$ Wasserstoff oder Niederalkyl bedeutet,

X, Y und Z ein N-Atom oder ein Ringglied $=\overset{R^3}{\overset{|}{C}}-$ bedeutet, wobei wenigstens einer der X, Y oder Z ein N-Atom bedeutet,

$R^3$ Wasserstoff, Halogen, Niederalkoxy, Niederalkylmercapto, Niederalkyl, Halogen-niederalkyl, Nitro, Cyan, Carbonsäureamid oder einen ankondensierten Phenyllenrest bedeutet, und

n eine ganze Zahl von 1 bis 4 bedeutet,

gegebenenfalls unter Verwendung von Hilfsstoffen und Trägerstoffen in eine geeignete Applikationsform überführt.